# EUROPÄISCHE PATENTSCHRIFT

(11) **EP 2 319 411 B1**
(45) Veröffentlichungstag und Bekanntmachung des Hinweises auf die Patenterteilung: **30.11.2016**
(21) Anmeldenummer: 10166212.0
(22) Anmeldetag: 23.10.2006
(51) Int. Cl.: A61B 5/05, A61B 5/107, A61B 5/02

(54) **Gerät zur elektrischen Messung von Körperfunktionen und Zuständen**
Device for electrical measurement of bodily functions and states.
Appareil pour la mesure électrique des fonctions du corps et des états

(30) Priorität: 21.10.2005 AT 17242005
(43) Veröffentlichungstag der Anmeldung: 11.05.2011
(62) Teilanmeldung aus: 06804359.5
(73) Patentinhaber: Skrabal, Falko, 8043 Graz (AT)
(72) Erfinder: Skrabal, Falko, 8043 Graz (AT)
(74) Vertreter: Margotti, Herwig Franz

(56) Entgegenhaltungen:
- WO-A1-2004/030535
- WO-A1-2004/032738
- WO-A1-2005/077260
- WO-A2-97/11638
- GHANEM ET AL: "Heart-Surface Reconstruction and ECG Electrodes Localization Using Fluoroscopy, Epipolar Geometry and Stereovision: Application to Noninvasive Imaging of Cardiac Electrical Activity", IEEE TRANSACTIONS ON MEDICAL IMAGING, Bd. 22, Nr. 10, Oktober 2003 (2003-10), Seiten 1307-1318, XP002420605,

## Beschreibung

Seit einem Jahrhundert wird jährlich bei Millionen Patienten die elektrische Aktivität des Herzens in Form eines EKGs aufgezeichnet. In der vorliegenden Patentschrift wird gezeigt, wie ohne nennenswerten Mehraufwand und in der selben Zeit mit dem selben Personal mit Hilfe eines sehr kostengünstigen Gerätes auch gleichzeitig eine komplette Aufzeichnung der mechanischen Aktivität des Herzens ("Function"), über arterielle und venöse Durchblutung der Extremitäten, ein Bild über die Körperzusammensetzung, Quantität der Flüssigkeit in den einzelnen Körperkompartmente ("Spaces") und damit ein sehr genaues Bild über die Körperhomöostase gewonnen werden kann. Wenn dies in der Routine ohne wesentliche Mehrkosten und ohne vermehrten Zeitaufwand geschehen kann, wird dieses neue Funktions-EKG das bisher rein elektrische EKG ersetzen:
Eines der hauptsächlichen ungelösten Probleme in der Betreuung von kranken, speziell kritisch kranken Menschen ist die Erfassung des Flüssigkeitshaushaltes, der Körperzusammensetzung und die haemodynamische Unterstützung. ("The art of fluid administration and haemodynamic support is one of the most challenging aspects of treating critically ill patients", Zitat aus E:W. Ely & G.R. Bernard in Transfusions in critically ill patients, Editorial NEJM 340: 467-468, 1999). Es gibt z.B. keine praktisch brauchbaren Methoden um Dehydration, Hyperhydration, Verlust von Extrazellulärvolumen, intrathorakalem Flüssigkeitsgehalt, Ascites, Intrazellulärvolumen usw. einfach in der Routine zu erfassen und damit die Therapie zu steuern. Darum sind Ärzte noch immer darauf angewiesen sich nach uralten und wenig verlässlichen Zeichen wie Hautturgor, Bulbushärte usw. zu richten. Auch Ultraschallmethoden wie Cava-Durchmesser sind wenig verlässlich und in vielen Fällen wird es notwendig einen Cava-katheter zur Messung des zentralen Venendruckes zu messen. Auch diese invasive Messung des zentralen Venendruckes ist speziell bei herzkranken Menschen wenig verlässlich um die Notwendigkeit von Flüssigkeitsersatz zu ermitteln. Andere Methoden wie einfache Impedanzverfahren haben sich zur Betreuung von kritisch kranken Menschen ebenfalls nicht bewährt, so ergibt eine medline Suche im Juli 2005 unter den Begriffen impedance, extracellular fluid volume und intensive care bei jeweils mehr als tausend Einzelzitaten nicht einen einzigen gemeinsamen Treffer und Impedanzverfahren werden auf Intensivstationen nicht routinemäßig verwendet. Die Schätzung von "fat free mass" bzw. Körperwasser und Körperfett durch Impedanzmessungen erfordert bisher die Berücksichtigung von Gewicht, Größe, Alter, Geschlecht die in die Schätzgleichungen eingehen. Ziel dieser Schätzparameter ist es das Absolutvolumen des Körpers zu schätzen, um dann auch die Teilvolumina "abschätzen" zu können. Bei Intensivpatienten oder Herz-und Nierenkranken, genügt es nicht zu schätzen, sondern es ist notwendig, die Detailvolumina bis auf einen Liter genau oder besser noch genauer zu erfassen, ganz abgesehen davon, dass es bei dieser Patientengruppe oft unmöglich oder sehr schwierig Größe und Gewicht zu ermitteln, da die Patienten bettlägerig sind. Außerdem weichen die mit Hilfe der Impedanz bestimmten Messgrößen bei verschiedenen Messungen am selben Patienten um bis zu 10 % voneinander ab. Ein Versuch, bei akutkranken chirurgischen Patienten die Änderung der Flüssigkeitsbilanz auf Grund von Mehrfrequenzbioimpedanz abzuschätzen, zeigte, dass die Methode zwar für eine Gruppe signifikante Ergebnisse zeigte, beim individuellen Patienten war die Methode ungeeignet, da sowohl negative als auch positive Korrelationen zwischen tatsächlichen Flüssigkeitsveränderungen und Impedanzergebnissen gefunden wurden. (Chiolero RL et al. Intensive Care Med. 1992;18(6):322-6). Eine andere Studie zeigte, dass Veränderungen der Flüssigkeitsbilanz nur dann durch Bioimpedanz erkannt werden konnten, wenn der Gewichtsunterschied mehr als 3 kg (und damit Liter) betrug!!! (Roos AN et al Crit Care Med. 1993 Jun;21(6):871-7.).

In der Medizin besteht auch häufig die Notwendigkeit der Messung der mechanischen Aktion des Herzens. So wird mittels verschiedener Verfahren und Gerät, wie z.B. Echokardiographie, mit oder ohne Farbdoppler die Schlagkraft, die Inotropie, die Kontraktilität und die Ejection Fraction gemessen. Weiters wird häufig die Menge des Blutes, das innerhalb eines Herzschlages vom Herz ausgeworfen wird, das Schlagvolumen und anderer haemodynamischer Parameter, ermittelt. Daraus lässt sich dann bei Kenntnis der Herzfrequenz das Herzminutenvolumen errechnen. Aus den erwähnten Größen lässt sich die Funktion des Herzens ableiten, es lässt sich die Diagnose von Herzkrankheiten stellen, es. können neue physiologische Erkenntnisse gewonnen werden. Jedoch kann die Überwachung von schwer herzkranken Patienten auf Intensivstationen oder während der Narkose mittels der Echokardiographie nicht wirklich erfolgen, weil ständig ein Untersucher da sein müsste. Wegen der Wichtigkeit dieses Problems gibt es auch zahlreiche weitere Methoden zur Messung des Herzzeitvolumens (HZV = Cardiac Output = CO) in der Medizin. So wird z.B. ein Katheter in die Arteria pulmonalis und/oder in die Aorta eingeführt und dort wird mittels einer Indikatorsubstanz, die z.B. Wärme, Kälte, Kochsalz oder Lithium sein kann, aus dem Abfall der Konzentration erwähnter Indikatorsubstanz mittels des Fick'schen Prinzips das Herzzeitvolumen (HZV) gemessen. Der Nachteil dieser Methode ist die Einführung eines Katheters in ein menschliches Gefäß mit allen daraus resultierenden Komplikationen, wie Blutung und Infektion.

In jüngster Zeit wurde versucht, auch das Fick'sche Prinzip für die Messung des Herzzeitvolumens durch Messung von Gasen in der Atemluft anzuwenden. Dies ist deswegen möglich, weil ein sehr rascher Gasaustausch zwischen Blut und Atemluft erfolgt, so dass man die Konzentrationen in diesen beiden Medien faktisch gleichsetzen kann. Wenn nun der Atemluft ein Gas beigemischt wird, erhöht sich dessen Konzentration auch im Blut, beendet man die Beisetzung des Gases, dann kommt es zu einem Abfall des Gases im Blut und auch in der Atemluft, wobei aus dem Abfall der Konzentration in der Zeiteinheit wiederum nach dem Fick'schen Prinzip das Herzzeitvolumen HZV gemessen werden kann. Eine Methode die sich dabei speziell bewährt hat, ist das CO₂ Wiederatmen, dabei wird eine Schleife (Loop) in die Atemwege des Patienten eingebracht und der Patient atmet eine bestimmte Zeit seine eigene ausgeatmete Luft wieder, so dass es zu einem Anstieg der CO2-Konzentration im Blut kommt. Der Nachteil dieser Methoden ist, dass der Patient mit einem Mundstück versehen werden muss und eine möglichst konstante Atmung erfolgen muss, damit eine gleichmäßige Konzentration der Atemgase in der Atemluft und im Blut gewährleistet wird. Darum wird dieses Verfahren und Gerät hauptsächlich in der Narkose mit konstantem Atemzugvolumen und konstanter Atemfrequenz angewendet. Ein weiteres Verfahren und Gerät besteht aus einer ähnlichen Methode, wobei statt des CO2 ein Mischung von inerten Gasen, welche inhaliert werden und das ebenfalls mit dem Blut rasch equilibriert, für die Messung des HZV verwendet wird.

Ein Gerät nach der Präambel von Anspruch 1 ist schon aus WO2005077260 bekannt.

Ein weiteres Verfahren und Gerät ist die Messung des Schlagvolumen und anderer haemodynamischer Parameters aus der Pulsform, die an einer peripheren Arterie abgenommen wird. Eine Änderung der Pulsform ist auch durch eine Änderung des Schlagvolumen und anderer haemodynamischer Parameters bedingt, woraus sich indirekt die Änderung des Schlagvolumen und anderer haemodynamischer Parameters über eine Transferfunktion ableiten lässt. Diese Methode muss am Anfang einmal mit einem der oben beschriebenen Verfahren und Gerät geeicht werden, sie ist außerdem nicht hinreichend genau. Eine weitere Methode ist das Messen einer Indikatorsubstanz wie z.B. Indigogrün transkutan an den Kapillaren des Ohres oder Finger, was die Genauigkeit des Fick'schen Prinzips sehr verringert.

Eine weitere Methode ist die Impedanzkardiographie (ICG). Bei dieser wird ein konstantes Wechselstromfeld an den Thorax angelegt, und die Änderung der Wechselspannung, die durch dieses Wechselstromfeld entsteht, weist auf eine Änderung des Flüssigkeitsgehaltes im Thorax hin. Genauer gesagt wird der Wechselstromwiderstand (Impedanz) mit dieser Methode gemessen, der ein Maß für die Änderung des thorakalen Flüssigkeitsgehaltes darstellt. Diese Änderung des Flüssigkeitsgehaltes im Thorax wiederum dient als Maß für die pro Schlag ausgeworfene Menge an Blut. Aus dem Schlagvolumen und anderer haemodynamischer Parameter (SV) und der Herzfrequenz (HR) lässt sich dann das Herzzeitvolumen (HZV = SV*HR) errechnen. Die Hauptprobleme bei der Impedanzkardiographie, wie sie derzeit noch immer verwendet wird, sind mehrfach: Um die Änderung des Flüssigkeitsvolumens im Thorax mit dem Herzschlag interpretieren zu können, müsste zuerst einmal der tatsächliche Flüssigkeitsgehalt des Thorax bekannt sein, was bisher mit der Impedanzmessung nur sehr schlecht gelungen ist. Weiter ist bisher nur sehr schlecht bekannt, durch welche Flüssigkeitsverschiebung, nämlich z.B. Auswurf in die Schlagader, Auswurf in die Lungenarterie, Verschiebung von Blut innerhalb der Lungengefäße usw. die Änderung des Impedanzsignals mit dem Herzschlag bedingt ist.

Üblicherweise wird für das ICG ein Elektrodenpaar, das den Strom zum Körper führt, an der oberen und an der unteren Thoraxbegrenzung angebracht. Innerhalb dieses Elektrodenpaares wird ein zweites Elektrodenpaar zur Messung der resultierenden Wechselspannung angebracht. Der Abstand zwischen beiden Elektroden ist also abhängig von der Länge des Thorax und wird als Elektrodenabstand in der weiteren Folge beschrieben. Bisher wurden für diesen Zweck entweder Zirkulärelektroden oder auch Punktelektroden, ähnlich wie EKG-Elektroden verwendet. In der Patentanmeldung "Medizinische Elektrode"[¹] wurde eine neue Elektrodenanordnung beschrieben, bei der auf der selben Folie über eine kurze Strecke, jeweils parallel zwei Bandelektroden angebracht sind, deren Abstand durch die gemeinsame Trägerfolie genau vorgegeben und reproduzierbar ist. Eine dieser parallel verlaufenden Bandelektroden, die auf dieser gemeinsamen Trägerfolie aufgebracht ist, dient für die Aufbringung des Messstromes, die andere parallel verlaufende Bandelektrode ist für die Ableitung der Messspannung vorgesehen. Das obere Elektrodenpaar kann z.B. am Nacken, die unteren Elektrodenpaare jeweils links und rechts an der unteren Thoraxapertur angebracht werden. Diese Elektrodenanordnung zeigt eine wesentlich bessere Reproduzierbarkeit der Ergebnisse als die früher verwendeten Ringelektroden bzw.. auch besser als die in der Patentanmeldung US 4.450,527 SRAMEK [²] beschriebenen Spotelektroden.
¹ FORTIN J, NESSLER B, NESSLER W, SKRABAL F: "Medizinische Elektrode", A 392/2001, KL. A61B, eingereicht am 13.03.2001, EP 1377 212 B1
² SRAMEK B: "Noninvasive Continuous Cardiac Output Anzeige-Anzeige-Monitor" US 4,450.527, 22. Mai 1984

Nachteile der beschriebenen ImpedanzVerfahren und Gerät waren bisher, dass die Ergebnisse entweder nach der KUBICEK-Gleichung [³, ⁴] bzw.. nach der SRAMEK-Gleichung [⁵, ⁶] errechnet wurden, die beide mit sehr vereinfachenden Annahmen über den menschlichen Körper hergeleitet wurden. In die erstere geht der an der Körperoberfläche gemessene Elektrodenabstand ein, in die zweite die Körpergröße. Diese Annahmen sind nur bedingt richtig, deshalb ergibt sich auch ein beträchtlicher Fehler in der Berechnung des Schlagvolumen und anderer haemodynamischer Parameters und des Herzzeitvolumens. Vor allem bei Herzkrankheiten mit niedrigem Schlagvolumen wird bei Einbringen von Gewicht und Größe in die Formel das Schlagvolumen überschätzt, weil Bias in Richtung normaler Werte erzeugt wird⁷. In letzter Zeit wurde die Impedanzmethode weiter "verbessert" indem zuletzt nicht nur die Körpergröße sondern auch das Gewicht in die Formel zur Schätzung des Schlagvolumens eingeht. Das heißt mit anderen Worten, dass das Ergebnis in erster Linie aus den Körpermaßen geschätzt wird. So erzielt man durch diesen Trick zwar bei Gesunden eine akzeptable Übereinstimmung mit den "Gold Standard" Methoden wie dem Fick'schen Prinzip bei Gesunden weil das Herzminutenvolumen zum gesunden Körper passt wie ein hautnah geschneiderter Anzug. Aber wen interessiert das Herzminutenvolumen bei Gesunden, das man auch ohne Messungen sehr genau aus Gewicht, Größe, sowie Geschlecht und Alter (als Maß für die stoffwechselaktive Körpermasse) schätzen kann⁸. Bei Abweichungen von der Norm wird damit das Verfahren gleichzeitig weniger brauchbar, weil bei Herzschwäche nicht mehr Größe und Gewicht des Menschen sondern die Zahl seiner kranken Herzmuskelfasern die Herzleistung bestimmen. In der Patentanmeldung PCT/AT 03/00302 wurde ein neues Verfahren und Gerät der Impedanzkardiographie beschrieben, bei dem mittels Messung an mehreren Orten und mit Hilfe von mehreren Frequenzen eine Verbesserung der Impedanzkardiographie erreicht werden konnte. Speziell wird dabei das im wesentlich selbe Segment bei zwei gering unterschiedlichen Längen gemessen. Speziell konnte daraus dann eine "operative Länge" errechnet werden, welche die Vorhersage des mit einem "Goldstandard" gemessenen Schlagvolumens bzw. Auswurffraktion verbessert. Diese Methode wurde als multi-site-frequency Elektromechanokardiographie (*_{msf}*ELMC) bezeichnet. Ein weiterer Fortschritt dieser Methode ist, dass nicht mehr Annahmen über die menschliche Geometrie gemacht und Modelle gebildet werden, sondern dass in einem "Black Box Approach" nur die elektrisch gemessenen Parameter in der Vorhersagegleichung verwendet werden, die in einer partiellen Regressionsanalyse statistisch signifikant und klinisch relevant zur Vorhersage beitragen.

Die Erfindung ist in Anspruch 1 definiert.
³ KUBICEK, W.G., I.N.KARNEGIS, R.P. PATTERSON, D.A. WITSOE, R.H. MATTSON: Development and evaluation of an impedance cardiac output system. Aerospace Medicine 37, 1208 - 1212 (1966*)*
⁴ KUBICEK, W.G., F.J. KOTTE, M.U. RAMOS, R.P. PATTERSON, D.A. WITSOE, J.W. LA BREE, W. REMOLE, T.E. LAYMAN, H. SCHOENING, D. SMITH: The minnesota impedance cardiograph - theory and applications. Biomed. Eng., 9, 410 - 416, (1974*)2*
⁵ SRAMEK, B: Noninvasive technique for measurement of cardiac output by means of electrical impedance. Proceedings of the Vth ICEBI Tokyo, (1981*)*
⁶ SRAMEK, B.BO, D.M. ROSE, A. MIYAMOTO: Stroke volume equation with a linear base impedance model and its accuracy, as compared to thermodilution and magnetic flowmeter techniques in humans and animals. Proceedings of the VIth ICEBI, Zadar, Yugoslavia, S. 38 (1983*)* ⁷ Skrabal F et al. Europ J Heart Failure Multisite Frequency Electromechanocardiography for the prediction of ejection fraction and stroke volume in heart failure 7: 974-83, 2005
⁸ Cohn JN et al. Hypertension 26: 503-508, 1995

In der gegenständlichen Anmeldung wird eine neue Methode zur Messung von Herzleistung (Function) und von Körperräumen (Spaces), das so genannte Function & Spaces *(FS)*-ECG beschrieben. Speziell wurden bei der gegenständlichen Anmeldung folgende Anmeldungen berücksichtigt und eine Abgrenzung gegenüber diesen hergestellt: In US 6015393 (Hovland) wird eine Methode zur Messung der penilen Tumeneszenz vorgestellt, bei der die gesamte Länge des Penis und ein kleines Segment des Penis separat vermessen wird, jedoch wird bei dieser Anmeldung nicht die gesamte Penislänge bei zwei nur gering unterschiedlichen Messabständen gemessen. In US 6339722 (Heethaar) wird am Thorax an einer unterschiedlichen Stelle die Thoraximpedanz ein zweites Mal gemessen um daraus die Verteilung intrazelluläres Wasser/Extrazelluläres Wasser zu ermitteln. In der Anmeldung DE 10249863A1 (Beise) wird mittels ICG und der Messung der Pulswelle versucht, eine kontinuierliche Blutdruckmessung zu verwirklichen, ähnliches wird auch in der Anmeldung EP1344489A1 (Medero) und in der Anmeldung EP 1344489 (Medero) beschrieben, wobei bei ersterer Anmeldung ein Pulsoxymeter, bei der Zweitären eine Impedanzmessung an einem unterschiedlichen Ort als dem Thorax verwendet wird, um aus der Änderung der Pulswellenlaufzeit eine kontinuierliche Blutdruckmessung zu erreichen. In der Anmeldung US 5642734 (Rubens) und in US 5526808 wird am Finger dasselbe Körpersegment bei zwei verschiedenen Frequenzen vermessen und daraus der Hämatokrit errechnet. Multiple Elektroden, die in den Körper eingeführt werden, sind auch in US 5109870 (Silny) zur Messung der Peristalsis des Darms und in US 4951682 (Petre) zur Messung des Cardiac Output mittels Einführung in das Herz beschrieben. Ein Training eines neuronalen Netzes zur Berechnung des CO wird in US 6186955 (Baura) beschrieben, wobei dieses Netz als integrierenden Bestandteil ein Trainingsset zum Lernen am individuellen Patienten und offensichtlich auch eine Goldstandardmessung als Referenz enthalten muss weil es offensichtlich nicht entsprechend für jeden einzelnen Patienten so verallgemeinern kann, dass es auch für den individuellen Patienten gültig ist..

In der gegenwärtigen Anmeldung wird ein System zur Messung von Flüssigkeit und Flüssigkeitsverschiebungen innerhalb des Körpers und seiner Segmente beschrieben, das eine komplette und automatische Analyse der Körperkompartmente, der Haemodynamik, sowie Flüssigkeitsverschiebungen innerhalb des Körpers ohne Anwesenheit eines Arztes und ohne Lernphase am individuellen Patienten ermöglicht. Es ist unter anderem beabsichtigt, dieses Verfahren und Gerät und Gerät z.B. mit einem Mehrkanal EKG zu verknüpfen, um so mit Hilfe des Function & Spaces (FS)- ECG im selben Arbeitsgang ohne Zeitverlust oder Mehraufwand auch eine komplette Flüssigkeits- und haemodynamische Analyse durchzuführen und dem Arzt einen Befund zu übermitteln, der nicht nur die elektrischen sondern auch die homeostatischen und haemodynamischen Störungen diagnostiziert. In der Analyse könnten unter anderem enthalten sein: Muskelmasse, Fettmasse, Extrazellulärvolumen, intrazelluläres Wasser, Ausmaß des Beinödems, Wasseransammlung im Bauch (Ascites), Wasseransammlung im Thorax (Lungenödem), extrazelluläres intrathorakales Flüssigkeitsvolumen, Pleuraergüsse mit Seitenlokalisation, arterielle Durchblutungsstörung der Beine mit Seitenlokalisation, Beinvenenthrombose oder venöse Insuffizienz mit Seitenlokalisation, weiters Auswurffraktion (= Ejection Fraction= EF), Schlagvolumen (SV), CO; Herzinsuffizienz NYHA Klasse I- IV, geschätzter Spiegel des natriuretischen Peptids (z.B. pro- BNP), , weiter Vorlast, Nachlast, Gefäßelastizität, Compliance der großen und kleinen Gefäße, Augmentationsindex, Blutdruck, Gefäßwiderstand, Barorezeptor-Reflexsensitivität, Tonus des autonomen Nervensystems, Vorlast des Herzens, kardiovaskuläre Prognose, wie Wahrscheinlichkeit von kardialen Ereignissen, , weiters Haematokrit und Serumnatriumkonzentration usw..

Dieses Gerät könnte dann als "Function&Spaces (*FS*)- EKG" , bezeichnet werden, weil es erstmals neben der elektrischen Funktion des Herzens auch die wichtigste Tätigkeit des Herzens, nämlich Aufrechterhaltung der Körperhomöostase und die Funktion als pulsatile Pumpe erfasst. "Wichtigste Tätigkeit" ist insofern der richtig gewählte Begriff, als die elektrische Funktion des Herzens weniger interessieren würde, solange nur die mechanische Funktion des Herzens optimal für die jeweilige Situation ist. Man hat sich nämlich bisher über ein Jahrhundert mit der Beurteilung der elektrischen Funktion des Herzens begnügen müssen, weil im selben Arbeitsgang die mechanische Aktion des Herzens nicht erfasst werden konnte.

Dieses Gerät soll selbstverständlich auch auf Überwachungsstationen zur laufenden Beurteilung des Patienten und zum Management des Patienten mit Herzkreislaufmedikamenten und zum Flüssigkeitsmanagement eingesetzt werden und wird auch in jeder Arztpraxis zumindest mit Teilkomponenten von Vorteil sein.

Damit eine Akzeptanz durch Arzt und Patienten gegeben ist, müssen mehrere Voraussetzungen erfüllt sein:
1. Muss die Untersuchung rasch und ohne Aufwand durchgeführt werden können, ideal wäre es diese im selben Arbeitsgang mit einer bereits auch ständig routinemäßig verwendeten Methode, wie z.B. dem EKG durchzuführen. Dies scheint deswegen notwendig, weil die Arbeitszeit des medizinischen Personals den größten Kostenfaktor in der Medizin darstellt und manche wertvolle Methoden nur deswegen in der Routine nicht Einzug finden, weil sie zusätzliches Personal beanspruchen würden. Für eine Praktikabilität sollte die Zahl der verwendeten Elektroden auf ein Mindestmaß reduziert werden.
2. Muss die Aussage der Methode von großem praktischem medizinischen Interesse und trotz der einfachen Durchführung sehr präzise und besonders gut reproduzierbar sein, damit sie im Alltag verwendet wird.

In der beschriebenen Anmeldung ist es mit nur sieben bis neun Doppel-bzw. Dreifach-Elektroden (weniger als für ein EKG) gleichzeitig mit einer kontaktlosen Messung des Körpervolumens und des Volumens seiner Segmente möglich, sehr präzise obige Parameter zu ermitteln.

Erfindungsgemäß werden diese Vorteile dadurch erreicht, dass während der Erfassung der elektrischen Parameter gleichzeitig und unbemerkt berührungslos ein dreidimensionales Abbild des untersuchten Körpers erzeugt wird, aus dem das tatsächliche Volumen des Gesamtkörpers und gleichzeitig auch seiner Segmente errechnet werden kann. Gleichzeitig wird durch das berührungslose Messgerät auch die genaue Lokalisation der Elektroden ermittelt, so dass der Elektrodenabstand und der Querschnitt des dazwischen liegenden Körpersegmentes auf jeder beliebigen Ebene dazwischen und damit auch das genaue Volumen des dazwischen liegenden Segmentes erfasst werden kann. Dann könnte man gleichzeitig z.B. das Extrazelluläre und Intrazelluläre Wasser nicht nur sehr viel genauer elektrisch messen sondern auch absolut in Litern und in % des Körpervolumens angeben, wie das der beste internationale Standard ist und gleichzeitig kann die Gewebsverteilung in allen Körpersegmenten erfasst werden..

Bei den Geräten zur Oberflächenvermessung und damit Volumenmessung könnte es sich z.B. um Distanzmessgeräte und eventuell zusätzlich Winkelmessgeräte handeln, wie sie aus der Technik bereits gut bekannt und wie sie kostengünstig serienfertig zu kaufen sind. Dabei könnte es sich z.B. um Radio- Ultraschall- oder optische Messgeräte handeln, die in bekannter Weise die physikalischen Eigenschaften des Körpers, wie das Volumen (das sich aus der dem Abstand der Oberfläche des Körpers zum Messgerät oder den Messgeräten , bzw. auch aus der Distanz der Körperteile im Verhältnis zur Distanz der Untersuchungsliege ergibt). Gleichzeitig kann so auch die genaue Position der am Körper angebrachten elektrischen Elektroden bzw. anderer Aufnehmer sowie auch die Distanzen zwischen den einzelnen Elektroden sowie Aufnehmer voneinander ermitteln werden. Dazu könnte das reflektierte oder gestreute Signal analysiert werden. Üblicherweise wird dafür entweder eine Interferometry, "Time-of Flight" oder eine Triangulations- Methode verwendet. Für die Triangulationsmethode eignet sich auch besonders das Infrarotlicht, das z.B. mit Hilfe von CCD oder CMOS den Einfallswinkel und damit die Distanz zum Objekt wiedergibt. Im einfachsten Fall genügt es sogar ein digitales Abbild des Patienten in mehr als einer Ebene herzustellen z.B. mit Hilfe von sogenannten Charge Coupled Devices (CCD) oder auch mit CMOS, um daraus mit speziellen Algorithmen das Volumen des Körpers und seiner Abschnitte zu errechen

Dafür könnte auch die (z.B. white light) "phase measurement profilometry (PMP)" Technik verwendet werden. Damit die Vorrichtung durch das umgebende Licht nicht gestört wird könnte auch ein "Infrared Grid Projektor" verwendet werden, der in Kombination mit billigen Digitalkameras verwendet werden kann.

Inzwischen gibt es auch sehr einfache Verfahren dazu, wie z.B. die Photogrammetrie, um aus digitalen Bildern ein dreidimensionales Bild des Körpers zu erzeugen und damit Oberfläche und Volumen zu errechnen (Appl Physiol Online). Die Technik ist auch im Detail unter dem Titel "stereo photographic digital topography" von Mikat R.P. (www.css.edu/users/tboone2/asep/Mikat.doc) beschrieben. Diese Methode kann z.B. auch mit der "reference point technique" kombiniert werden, bei der ein Muster von Linien bzw. Punkten auf den Körper projiziert wird, aus deren Verzerrung dann sehr genau das dreidimensionale Bild rekonstruiert werden kann. Eine gute Übersicht über einige schon ältere mögliche Verfahren ist in HERRON, R.E. Biostereometric measurement of body form. Yearbook of Anthropometry, 16:80-121, 1972 gegeben. Auch an die Verwendung anderer zukünftig kommenden Methoden zur Oberflächenerfassung bzw. zur Volumenmessung von Körpersegmenten ist selbstverständlich gedacht. Es kann z.B. auch günstig sein, gemeinsam mit dem untersuchten Körper einen bekannten Maßstab mit abzubilden, um so wechselnde Entfernungen und Winkeln zwischen den Geräten zur berührungslosen Oberflächenmessung und dem Körper zu korrigieren. Diese wechselnden Entfernungen und Winkeln entstehen vor allem bei Verwendung eines mobilen Messplatzes, der jeweils zum untersuchten Patienten geschoben wird.

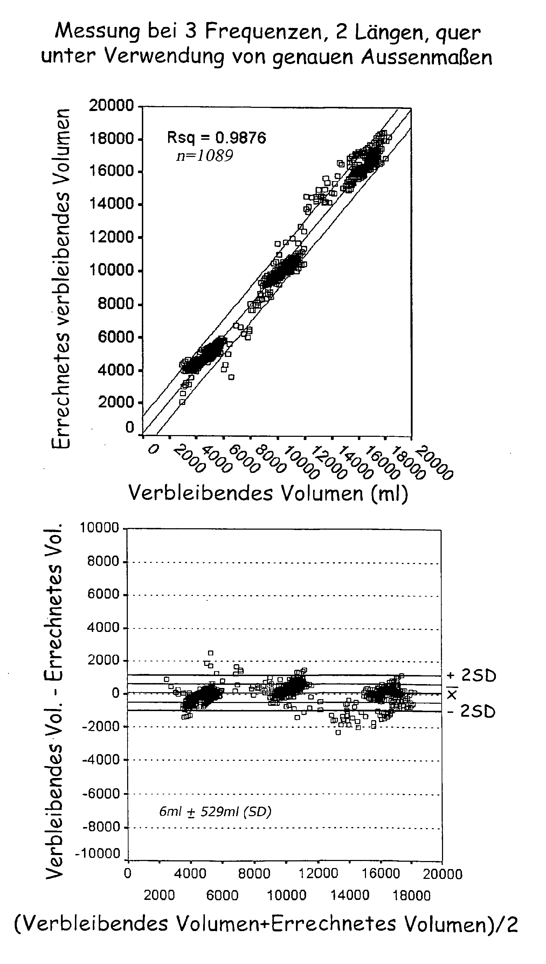

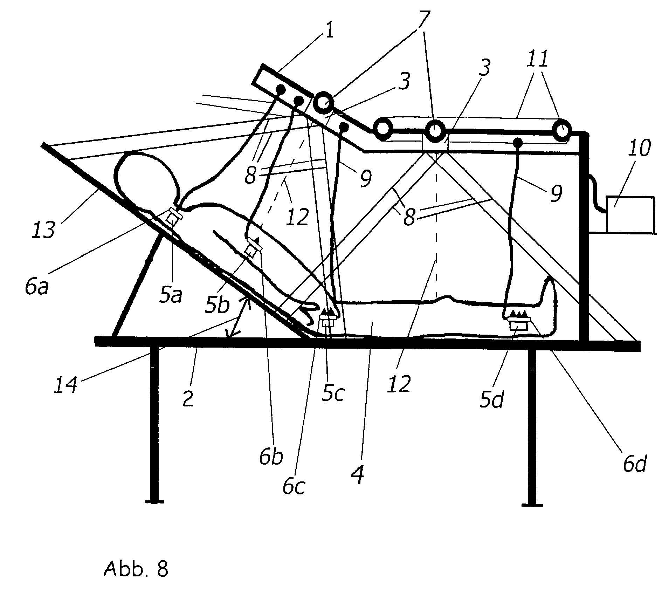

Als weiteres verwendbares kontaktloses Verfahren wird z.B. auch die "Verdrängungsmethode" angeführt, wo z.B. der untersuchte Körper in einem abgeschlossenen Raum platziert wird und die Verdrängung bzw. nach dem Boyle'schen Gesetz die Kompression der verdrängten Luft gemessen werden kann. Diese alternative Technik ist unter anderem deswegen nicht so günstig, weil es nicht einfach ist, das Volumen von einzelnen Körperteilen separat zu erfassen. Mit den hier beschriebenen berührungslosen Verfahren ist es gleichzeitig auch möglich die korrekte Lage der Elektroden zu erfassen, indem die Elektroden oder deren Elektrodenhalter durch charakteristische Profilgebung oder andere Eigenschaftsgebung, wie z.B. Reflexion, Farbgebung oder Emission z.B. von elektromagnetischen oder optischen Wellen, genau in dem Bereich, in dem die verwendete Sensoreinrichtung empfindlich ist, von den berührungslos arbeitenden Oberflächenmessgeräten erkannt werden können. Bei Kenntnis der Distanz und des Winkels von den einzelnen Elektroden zum Distanzhalter kann sofort die direkte Distanz zwischen den Elektroden ermittelt werden. Die wahre Distanz zwischen den Elektroden ist deswegen wichtig zu erkennen, weil nur bei Kenntnis der wahren Distanz bzw., des wahren zwischen den Elektroden liegenden Volumens, eine etwaige Fehlhydrierung in diesem Körpersegment erkannt werden kann (siehe Abb. 7 und Abb. 8).. Besonders vorteilhaft zur Entdeckung der Elektrodenposition sind dabei Elektrodenanschlüssen die auf Grund von speziellen physikalischen Eigenschaften (z.B.. Profilgebung, Farbe, einer speziellen Reflexion, Schwingung, Temperatur usw.) als eindeutiges Ziel des Distanz- und Winkelmessgerätes bzw. des digitalen Bildes identifiziert werden können. Damit könnte dann auch sofort eine fehlerhafte Anbringung oder Vertauschung der Elektroden erkannt und dem Benutzer signalisiert werden, wenn die einzelnen Elektroden eine jeweils spezielle Kennung aufweisen. Auch könnte mit den Distanzmessgeräten der auf der Untersuchungsliege liegende Patient genau dreidimensional vermessen werden. Durch das dreidimensionale Abbild des Patienten können dann sehr einfach die Volumina der einzelnen Segmente ermittelt werden und die Fehlhydrierung und der Fettanteil in den einzelnen Kompartments erstmals genau genug für die klinische Diagnostik ermittelt werden. Erfindungsgemäß werden diese Vorteile weiters dadurch erreicht, dass die Impedanz und deren Änderung mit der Zeit von verschiedenen annähernd zylindrischen oder ovalen Körpersegmenten mit relativ konstantem Querschnitt entweder sequentiell oder gleichzeitig an verschiedenen nachgeschalteten Körpersegmenten und dies in mehr als einer Richtung gemessen wird. Die Messung an verschiedenen nachgeschalteten Körpersegmenten hat den Vorteil, dass die Geschwindigkeit der Volumenwelle erkannt werden kann, aus der dann abgeleitet werden kann, ob und wie viel die reflektierte Volumenwelle zur Änderung des Volumens während der Herzaktion im Thorax beiträgt. Bisher wurde nämlich nie beachtet, dass die Änderung des Volumens im Thorax (gemessen zB. als dz oder dz/dt der Thoraximpedanz z) nicht nur durch die Herzaktion selbst, sondern durch eine Reflexion der Volumenwelle an den Gefäßen in der Peripherie zustande kommen kann.

In mehr als einer Richtung heißt, dass zusätzlich zur bisher verwendeten Längsrichtung das jeweilige Körpersegment auch quer und/oder diagonal vermessen wird, wobei vorteilhafter Weise das Prinzip der 4-Punktmessung beibehalten wird. Für die Vermessung der Zylindersegmente im Querdurchmesser ist zwar eine echte Vierpunktmessung mit außerhalb der Spannungselektroden liegenden Stromelektroden zwar nicht möglich, es kann jedoch die jeweilige Stromelektrode neben der Spannungselektrode liegen. Auch an eine Zweipunktmessung mit Ausführung der jeweiligen Elektrode als Strom und Spannungselektrode ist gedacht, wobei so auch der Widerstand und damit der Flüssigkeitsgehalt der Haut erfasst werden kann.

Für die Impedanzmessung in Längsrichtung wird die Einspeisung wie üblich außerhalb der Spannungsmessungspunkte, z.B. also am Kopf oder in Kopfnähe einerseits und an der unteren Thoraxapertur oder am Abdomen oder am Bein oder am Fuß oder in Fußnähe andererseits erfolgen, die Abgreifpunkte für die Spannungsmessung könnten den Körper z.B. in ein Thoraxsegment, ein Abdomensegment und in zumindest jeweils ein Beinsegment oder mehrere Beinsegmente zerlegen, gegebenenfalls könnte auch zumindest ein Armsegment oder mehrere Armsegmente gemessen werden, in welchem Fall der Strom distal davon an der Hand oder in Handnähe einzubringen ist. Dies ist als segmentale Impedanzmessung an und für sich schon lange bekannt.

Weiters wird vorgeschlagen, linkes und rechtes Bein, bzw.. auch linken und rechten Arm separat zu vermessen um so auch arterielle und/oder venöse Durchblutungsstörungen an den Extremitäten erkennen zu können.

Die Vermessung sowohl in Längs- als auch in Quer- und oder Diagonalrichtung weist folgende Vorteile auf: Um eine genaue Volumenmessung durchführen zu können, muss sowohl der elektrisch partizipierende Durchmesser des Zylinders (oder Elipsoids, bzw. unregelmäßig geformter Fläche) einerseits, als auch der elektrisch partizipierenden Länge des zylindrischen oder ovalen Segmentes bekannt sein. Mit der Anmeldung PCT/AT03/00302 , bei die Impedanz über die Länge des Zylinders bei zwei sehr ähnlichen Segmentlängen gemessen wird, ist es zwar möglich eine operative Länge zu ermitteln: diese gibt eine virtuelle Länge wieder, die durch elektrische Bäuche verkürzt bzw. durch elektrische Taillen verlängert wird. Sie entspricht damit einem **gemeinsamen** Maß das Länge (L) /Querschnitt(A). Der Querschnitt A allein kann daraus nicht abgeleitet werden. Durch die Impedanzmessung quer über die Fläche des Kreis-oder ovalen Segmentes kann hingegen die elektrisch partizipierende Fläche A genau geschätzt werden. Dies ist wesentlich günstiger als den Querdurchmesser mit Hilfe einer Messung des Umfanges des Segmentes und der Formel ΔV= (C².L/(4π·z₀)). Δz zu schätzen, wie das Kubicek vorgeschlagen hat⁹.
⁹ Kubicek WG et al "The Minnesota Impedance Cardiograph-Theory and Applications" Biomed Engin September 9: 1974, pp 410-417

Diese Formel funktioniert nämlich in erster Linie zur Abschätzung einer Änderung des Volumens nicht jedoch zur Ermittlung des Absolutvolumens.

Bei Kenntnis des Querschnittes A und von L/A sind dann die wahren "elektrisch partizipierenden" Dimensionen des Zylinders oder Ovaloids bekannt, sodass dann auch die relativen Änderungen des Volumens durch Messung der Impedanzänderung dz/dt sowohl in Längs- als auch in Querrichtung sehr genau erfasst werden können. Um für die Quermessung die Vierpunktmethode anwenden zu können, nämlich auch hier die Trennung von Strom und Spannungselektroden beizubehalten, wird beispielsweise vorgeschlagen, die für die Spannungsmessung in Längsrichtung verwendeten Elektroden als Doppelelektroden auszuführen. In Längsrichtung können dann beide Teile der Doppelektrode (z.B. Band oder Spotelektroden) z.B. als Spannungselektrode verwendet werden, um zum Beispiel auch die operative Länge zu errechnen, wie das in der Patentanmeldung PCT/AT03/00302 vorgeschlagen wird. Bei der Quermessung kann jedoch eine der beiden Teile der Doppelelektrode als Strom-, der andere als Spannungselektrode verwendet werden, wie dies später an Hand der Abbildungen genau beschrieben wird. Ein weiterer Vorteil ist, dass bei der Quermessung besonders gut die Atemaktivität, nämlich Inspiration und Exspiration erkannt werden können. Die Erkennung von Inspiration und Exspiration ist u.a. auch deswegen wichtig, weil sich damit das Schlagvolumen ändert und die Änderung des Schlagvolumens mit Inspiration und Exspiration in der Intensivmedizin ein gutes Maß für den Flüssigkeitsbedarf des Organismus ("fluid responsiveness")darstellt. Genauso könnten natürlich auch separate Stromelektroden oder auch Spannungselektroden für die Quervermessung verwendet werden, was den Elektrodenaufwand und das Handling aufwendiger machen würde.

Obwohl es sehr kompliziert klingt, alle unterschiedlichen Körpersegmente sowohl Längs- als auch Quer oder auch Diagonal zu vermessen, ist dies in der Praxis mit nur 7 Elektroden sehr leicht möglich, die zum Teil oder komplett gleichzeitig auch als EKG-Elektroden verwendet werden können, sodass der Aufwand tatsächlich nicht größer als für das EKG allein ist. Damit unterscheidet sich auch die Methode wesentlich von der Impedanztomographie. Es ist natürlich innerhalb dieser Patentanmeldung durchaus bedacht, dass das Minimum an Elektroden durch Verwendung einer größeren Zahl an Elektroden noch genauer wird, wobei die Methode allerdings an Eleganz verliert.

Eine Eichung der vorgeschlagenen Impedanzmethode mit einer Goldstandardmethode, wie z.B. der DXA, Echokardiographie, MRI oder anderen Methoden ist sehr leicht möglich.

Auch eine Diagonalvermessung des untersuchten Segmentes ist sehr leicht möglich und kann wertvolle zusätzliche Information bringen, ohne dass am Körper neue Elektroden angebracht werden müssen. So können damit z.B. am Thorax ein- oder zweiseitige Pleuraergüsse erkannt werden. Ebenso das besonders in der Intensivmedizin so relevante extrazelluläre Lungenwasser. Hier bewährt sich auch besonders die separate Vermessung jeweils an einer Thoraxseite mit Hilfe von dort aufgebrachten Doppelelektroden und die Einspeisung des Stromes einerseits weiter kranial (Hals oder Kopf) und andererseits weiter kaudal (irgendwo kaudal vom Thorax).

Es wird vorgeschlagen, obige Messungen bei mehreren oder vielen Frequenzen bzw. auch einen kompletten Frequenz-Sweep durchzuführen. Speziell sind dafür zumindest zwei, besser vier Frequenzen von Interesse: a) zumindest 2 Frequenzen die in erster den Extrazellulärraum durchdringen in der Größenordnung zwischen 0.1 und ca. 40 kHz liegen (z.B. 0,5 und 1 bis 5kHz) und b) zumindest zwei weitere Frequenzen, die auch die Zellmembran durchdringen und so das Gesamtkörperwasser erfassen, in der Größenordnung zwischen größer als 40 kHz und 2 mHz, konkret z.B. 200 kHz und 400 kHz. Der Grund für jedes der beiden obigen Körperkompartmente mehrere, zumindest jedoch zwei Frequenzen zu wählen, ist folgender: Die Stromverteilung im Organismus ist durch die unterschiedliche Zusammensetzung der Gewebe äußerst inhomogen. In der Nachbarschaft von nicht- oder schlecht leitenden Strukturen kommt es im Rahmen von geringerer Interaktion der Feldlinien zu einer Verdichtung der Feldlinien, da in den Randzonen eine geringere Interaktion der Feldlinien stattfindet. Dieser Effekt ist umso ausgeprägter je höher die Frequenz des Wechselstromes. Mit der Messung von zwei Spannungen für das jeweilige Kompartment, lassen sich so besser die Inhomogenitäten des eines Kompartments (Extrazellulärraum z.B. bei 0,5 und 1-5 kHz) und des anderen Kompartments (Gesamtkörperwasser z.B. bei 200 und 400 kHz) erfassen. Die Auswirkung der Messung bei mehreren Frequenzen für nur ein Kompartment und der Einfluss einer hochpräzisen Vermessung der äußeren Dimensionen des Kompartments wird später an Hand von Phantomversuchen demonstriert. Auch eine separate Messung von "Resistance and Reactance und Bestimmung des "phase angle" ist gedacht, da diese Parameter unter anderem sehr guten Einblick in die Masse und funktionelle Integrität der zellulären Masse geben.

Die Wichtigkeit der genauen Vermessung der Kompartments speziell von schwer kranken Patienten ist in vielen Ursachen begründet. So trivial dies klingt, ist doch zu beachten, dass viele Intensivpatienten nicht gewogen werden können, was insbesondere wegen der nicht kontrollierbaren "insensible losses" durch Atmung, durch Flüssigkeitsverlust über die Haut, aber auch durch die schwer erfassbaren Verlust z.B. über den Stuhl das Flüssigkeitsmanagement äußerst problematisch macht. Auch genaue Bilanzstudien mit Messung aller Ausscheidungen über Haut und Atmung , Harn und Stuhl stimmt in keiner Weise mit dem gemessenen Körpergewicht zusammen (siehe z.B. Roos AN, Critical Care Medicine 21, 871-77, 1993).. Speziell ist natürlich die Verteilung des Körperwassers auf den Extrazellulärraum und Intrazellulärraum wichtig, da sich danach die Therapie richten muss. Bei Kenntnis des intra- und extrazellulären Wassers bzw. bei Kenntnis des Defizits kann speziell bei Eingabe des Serumnatriums auch die genaue Fehlmenge bzw. Überschuss an Natrium und Chlorid (für den Extrazellulärraum) und die Fehlmenge bzw. Überschuss an Kalium und Phosphat (für den Intrazellulärraum) völlig automatisch ermittelt werden, was dem Arzt die Substitutionstherapie sehr erleichtert. In Kombination mit einem EKG ergeben sich darüber hinaus auch erstmals entscheidende Vorteil in der Erkennung von Minderdurchblutungen des Herzmuskels. In der Ischämie (Minderdurchblutung des Herzmuskels) wird nämlich das Myokard steifer, die Pumpleistung sinkt und in der Lunge kommt es vorübergehend zu einer Flüssigkeitsstauung. Wenn also bei unklaren Herzbeschwerden des Patienten wie bisher ein EKG angefertigt wird, um Änderungen der Nachschwankung zu erkennen (die leider oft fehlen), erkennt man jetzt erstmals die (oft vorübergehende kurzzeitige) Erhöhung des intrathorakalen Volumens mit der gleichzeitigen Abnahme der Herzleistung.. Damit wird das EKG in der Ischämie Entdeckung viel sensitiver. Bei Verwendung des fixen Elektrodenabstandes zwischen zwei Elektroden kann auch der Widerstand zwischen beiden Elektroden und damit die Hautdurchblutung und der Flüssigkeitsgehalt der Haut ausgegeben werden, was ebenfalls die Flüssigkeitstherapie erleichtert. Dabei kann mit einer zusätzlicher Gleichstrommessung (z.B. mit einer Wheatstone'schen Brückenschaltung oder ähnlichem) besonders der Flüssigkeitsgehalt der oberflächlichen Schichten des cornum strateum gemessen werden, mit Wechselstrommessung der Flüssigkeitsgehalt von oberflächlichen und tiefen Hautschichten und über eine Kapazitätsmessung der Flüssigkeitsgehalt der tiefen Schichten des Cornum strateum. (Triebskorn A et al Dematologia 1983, 167: 64-9). Für diese Messung des Hautwiderstandes könnten auch die Gel-beschichteten EKG Elektroden herangezogen werden. Auch hier kann sich die Mehrfrequenzmessung Vorteile bringen (Janitzki AS, Vedder N, Multichannel measurements of skin resistance. Biomed Tech 1987 May;32(5):98-107). Diese Messung des Flüssigkeitsgehaltes der Haut bringt zusätzliche wichtige Information über den Flüssigkeitshaushalt des Menschen.

Weiter wird vorgeschlagen, auch Parameter für die Gefäßfunktion, speziell Arterienfunktion und Venenfunktion, bzw. auch Parameter für die Kongestion in den Körpersegmenten zusätzlich mit für die Berechnung der Herzfunktion heranzuziehen, da die Gefäßfunktion entscheidend die Herzfunktion mitbeeinflusst und damit auch die gemessene Impedanzkurve in nicht vorherzusehender Weise verfälschen kann, wenn die Arterienfunktion nicht mathematisch mitberücksichtigt wird. Als Parameter zur Korrektur der gemessenen Herzfunktionen kommt z.B. der Blutdruck, die Pulswellenlaufzeit, die Compliance der großen und kleineren Arterien, bzw. auch der Augmentationsindex¹⁰ in Frage. Besonders wichtig ist die laufende Kontrolle des zentralen Aortendruckes, der aus der Radialispulswelle genau berechnet werden kann. Von der Geburt bis zum Tod "sieht" das Herz nie den in der Medizin gemessenen Brachialis- oder Radialis-druck, sondern immer nur den Druck in der zentralen Aorta. Obwohl dies die Nachlast des Herzens bestimmt und obwohl dies seit vielen Jahren bekannt ist, wird der Parameter nicht in der Routine gemessen, weil dafür zusätzliches Personal notwendig wäre, das nicht vorhanden ist. Bisher ist dies nämlich zeitaufwendig, weil eine mechanische Vorrichtung händisch genau auf die Arterie justiert werden muss. In Kombination mit dem früher von Skrabal beschriebenen "Fluid Coupling"(Patent A 391.262, Skrabal) braucht nur eine Handgelenksmanschette ohne jede Justierung um das Handgelenk angelegt werden und der zentrale Aortendruck kann routinemäßig mit jedem *FS*-ECG ausgegeben werden. Auch dies ist für die Ischämieerkennung von Vorteil, weil vasokonstriktorische Substanzen, in der Ischämie produziert, den zentralen Aortendruck verändern. Weiters wurde bei invasiven Untersuchungen gezeigt, dass bei sehr rascher Pulswellenlaufzeit, die Pulswelle sehr früh in der Peripherie anlangt und damit so früh wieder zur zentralen Aorta reflektiert wird, dass die Reflexionswelle noch bei offener Aortenklappe am Herzen ankommt, und damit direkt die Nachlast des Herzens verstärkt. Was bisher nicht bedacht wurde, dass damit natürlich auch die Volumenwelle sehr rasch in die Peripherie und wieder zurück gelangen kann, was eine Erhöhung der Volumenwelle im Thorax und damit eine fälschliche Erhöhung des dz/dt verursachen kann. Bei Messung der Thoraximpedanz allein wird jedoch ausschließlich die Volumenzunahme im Thorax während der mechanischen Herzaktion registriert und daraus die Herzleistung wie z.B. Schlagvolumen bzw.. Auswurffraktion oder andere Parameter falsch geschätzt.
¹⁰ Rourke MF and Gallagher DE. J Hypert 14 (suppl5) S147-157, 1996

Ist die Nachlast durch eine ungünstige Gefäßfunktion erhöht, hat das direkte Konsequenzen für die Volumenveränderung und damit die Leitfähigkeit oder Impedanz im Thorax. So würde z.B. durch eine frühe Reflexion der Pulswelle bzw. "Volumenwelle" zurück in den Thorax das dz verfälscht werden. Ein Teil des dz stammt somit nicht von der Aktion des Herzens, sondern ein Teil stammt von der Reflexion der Pulswelle aus der Peripherie. Damit ist dies ein weiterer Faktor für die bisherige Ungenauigkeit der Impedanzkardiographie. So wurde z.B. auch gezeigt, dass das Ersetzen der zentralen Aorta mit ihrer Windkesselfunktion durch ein starres Glasrohr unmittelbar zu einer Reduktion der Auswurffraktion führt¹¹. Verwendet man konventionelle Methoden zur Messung der Herzleistung, wie z.B. die Auswurffraktion (=Ejection Fraction, EF), oder das Schlagvolumen, gemessen mit Hilfe der Echokardiographie, Isotopenmethoden, CT oder Magnetresonanz oder Fick'schem Prinzip bei denen die tatsächliche EF oder das tatsächliche Schlagvolumen gemessen und nicht geschätzt wird, ist das Ergebnis der Messung durch Änderungen der Nachlast unbeeinflusst und es wird immer das richtige Ergebnis ausgegeben. Bei der Impedanzkardiographie hingegen kann durch Änderungen der Nachlast oder des Gefäßzustandes das Ergebnis in die umgekehrte Richtung verfälscht werden. Eine Erhöhung der Nachlast bewirkt dann eine stärkere oder raschere Volumenzunahme im Thorax, aus welcher bei Zunahme ja bei der derzeitigen Impedanzkardiographie eine bessere Herzleistung abgeleitet wird, wobei das Gegenteil der Fall ist. So wird den Herzkranken durch eine allfällige frühere Reflexion der Pulswelle ein "verfälschter" rascherer und größerer Anstieg der Änderung der Impedanz mit dem Herzschlag und damit ein fälschlich höheres Schlagvolumen bzw. auch eine fälschlich höhere Auswurffraktion zugewiesen. Gerade die Compliance der Aorta und der großen Gefäße und die Pulswellenlaufzeit lässt sich jedoch sehr gut mit einfachen Methoden, wie z.B. aus einer Pulswellenanalyse sehr gut abschätzen, wobei z.B. auf die Methode von Watt und Burrus¹² verwiesen wird. Auch die distale Compliance der kleineren Gefäße hat einen Einfluss auf die Herzfunktion, da bei schlechter Compliance dieser Gefäße die Reflexionszone nach proximal verlagert wird, so dass ebenfalls eine frühe Reflexion und damit eine Augmentation des Aortendruckes erreicht wird, was die Impedanzkurve ebenfalls verändert. Auch die distale Compliance lässt sich mit der Methode von Watt und Burrus sehr gut abschätzen. Wobei nicht wie bei Watt und Burrus oder auch bei den Arbeiten von Cohn und Finkelstein das Herzminutenvolumen geschätzt werden braucht, sondern ja gleichzeitig genau gemessen wird, was die Methode wesentlich verbessert. Eine weitere Methode zur Messung der Augmentation des Aortendruckes durch geänderte Arterienfunktion stellt die Messung des Augmentationsindex¹³ dar, welcher ebenfalls aus der Pulswelle ermittelt wird.
¹¹ Urschel CW & Braunwald E: Am J Physiol 214: 298-304, 1969
¹² Watt TB und Burrus C, J Appl Physiol 40, 171-176, 1976

Für die Messung der Pulswellenlaufzeit kann in bekannter Weise ein druckempfindlicher Sensor an einer distalen Arterie oder auch eine Impedanzmessung an einem distalen Körperabschnitt, bzw. auch eine plethysmographische Methode an einem distalen Körperabschnitt bzw. auch eine transkutane Messung der Blutgase, wie z.B. eine Pulsoxymetrie, bzw. auch eine Flussmessung durch Dopplermethode usw. verwendet werden.

Für die Formanalyse der Pulswelle kann z.B. jeder über einer Arterie angebrachte Drucksensor, z.B. auch der in der Anmeldung US 6669648 verwendete Sensor mit flüssigkeitsgefüllter Blase verwendet werden. Dieser letztere ist deswegen vorteilhaft, weil er ohne jede Justierung durch medizinisches Personal zu einer sehr guten Formanalyse beiträgt.

Die Messung einer zweiten Impedanzkurve (dz oder dz/dt) an einem zweiten vom Herzen entfernten Ort kann ebenfalls dazu herangezogen werden und hat darüber hinaus viele weitere Vorteile. Erstens kann daraus die Pulswellenlaufzeit bzw.. die Veränderung der Volumenlaufzeit (unabhängig vom wahren Flüssigkeitstransport) als Maß für die Verfälschung der Impedanzkurve am Thorax abgelesen werden, außerdem können auch aus der Formveränderung des dz bzw. dz/dt in der Peripherie gegenüber dem dz bzw. dz/dt am Thorax weitere Rückschlüsse auf die Herzfunktion, speziell Schlagvolumen und Auswurffraktion, NYHA Klasse, BNP Spiegel usw. gezogen werden und so diese Parameter viel besser geschätzt werden, als bei alleiniger Messung der Impedanzänderung am Thorax. Ein weiterer Vorteil ergibt sich bei der Anwendung folgender Beobachtung: Es ist äußerst schwierig den Flüssigkeitsbedarf von Intensivpatienten abzuschätzen. Der pulmonalarterielle Verschlussdruck (pulmonary wedge pressure, PWP) hat sich dazu nämlich sehr schlecht bewährt. Es hat sich jedoch gezeigt, dass die Variation des systolischen Blutdruckes, bzw. der Blutdruckamplitude mit der Atmung ein sehr gutes Maß für den Flüssigkeitsbedarf des Organismus ist. Dies besonders bei beatmeten Patienten. Kommt es während der Beatmung zu einer geringen Variation des systolischen Blutdruckes oder des Pulsdruckes (< ungefähr 10%) ist eine weitere Flüssigkeitsgabe nicht von einer Erhöhung der Auswurfleistung des Herzens gefolgt und damit sinnlos und gefährlich. Dies wird in der Intensivmedizin als "Fluid Responsiveness" bezeichnet. Somit kann bei genauer Detektion der Atmung (z.B. durch die Quermessung der Impedanz am Thorax, wie oben beschrieben) einerseits automatisch die Atmung erfasst werden, und andererseits am Beinsegment eine Variation der Auswurfleistung des Herzens mit der Atmung über das am Bein gemessene dz/dt erfasst werden. Am Bein zu messen hat den Vorteil, dass im Gegensatz zum Thorax das dz/dt dort nur von der Herztätigkeit und nicht wie am Thorax vom Flüssigkeitsgehalt im Thorax mit Atmung, Änderung der Lungendurchblutung, Luftgehalt, Durchmesser usw. beeinflusst wird. Somit kann man erstmals ohne mechanische Abnehmer und damit artefaktfrei den Infusionsbedarf bei schwer kranken Patienten erkennen. Natürlich kann auch konventionell die Pulswelle z.B. mit Hilfe des "fluid coupling" (US 6669648) oder die vascular unloading technique (siehe Anmeldung US 6669648) herangezogen werden. Ein weiterer Vorteil ist, dass bei separater Messung von jeweils linkem oder rechtem Bein auch automatisch eine einseitige arterielle und venöse Durchblutungsstörung erkannt werden kann, weil sich bei Unterschieden der Durchblutung auch Unterschiede in der Volumenänderung mit der Herzaktion ergeben. Zur Erfassung von venösen Durchblutungsstörungen kann es sich dabei auch günstig erweisen, jeweils eine Staubinde an den Extremitäten anzubringen und intermittierend in diesen den Druck zu erhöhen und zu verringern, um so noch besser den venösen Abfluss in bekannter Weise mit Hilfe der Venenokklusionsplethysmographie abschätzen zu können.

Weder die Messung der Pulswellenlaufzeit noch der "Volumenlaufzeit" noch die Pulsoximetrie können jedoch über den tatsächlichen Transport von Blut von einem zum anderen Ort Auskunft geben. Es ist jedoch die Veränderung der Kreislaufzeit, speziell eine Verlangsamung des Bluttransportes, die für die Herzinsuffizienz charakteristisch ist. Diese ist nur dann erkenntlich, wenn tatsächlich der Transport eines Bolus von Blut vom Ort A nach dem Ort B gemessen werden kann. Dies kommt dem Fick'schen Prinzip gleich. In der gegenwärtigen Anmeldung wird vorgeschlagen eventuell zusätzlich auch einen elektrisch von der Körperoberfläche her erkenntlichen Bolus einer geänderten Leitfähigkeit des Blutes in einem oder mehreren verschiedenen Körpersegmenten zu messen. Dazu muss z.B. eine Substanz in den Kreislauf eingebracht werden, welche die Leitfähigkeit des Blutes vorübergehend verändert. Es könnte sich beispielsweise um eine Injektion von gegenüber dem Blutplasma hypertoner oder hypotoner Elektrolytlösung, speziell Kochsalzlösung, eventuell auch einer isotonen Elektrolytlösung handeln. Dieser Bolus erzeugt z.B. zuerst im Thorax oder einem Thoraxsegment eine vorübergehende Impedanzänderung, und mit Verzögerung in den folgenden Segmenten, z.B. einem nachgeschaltetem Thoraxsegment, oder im Abdomen und den Beinen ebenfalls eine Impedanzänderung. Aus der Impedanzänderung in einem Segment, das bevorzugt schmal ist, bzw. aus der Verzögerung des Impedanzsprunges von einem Segment zu einem nachgeschaltetem Segment können wichtige Rückschlüsse auf die Kreislauffunktion gezogen werden und auch EF und SV errechnet werden. Dabei hilft auch die Kenntnis der Elektrodenlage durch die berührungslose Entfernungsmessung wie oben beschrieben, weil so die zurückgelegte Wegstrecke des Bolus vermessen werden kann. Dazu können auch die Gleichungen, wie sie aus dem Fick'schen Prinzip z.B. aus der Thermodilution bekannten sind, verwendet werden. Eine isotone Elektrolyt-Lösung ist deswegen verwendbar, weil durch die isotone Lösung , die keine geformten Elemente wie rote Blutkörperchen enthält, gegenüber dem Vollblut ebenfalls eine Impedanzänderung erzielt wird, die gemessen werden kann, speziell dann, wenn dafür mehr als eine Frequenz verwendet wird, weil die Leitfähigkeit des Blutes bei unterschiedlichen Frequenzen deutlich unterschiedlich ist, je nachdem die Erythrozytenmembran vom Wechselstrom durchdrungen wird oder nicht.

Eine Grundvoraussetzung für die Methode ist, dass diese möglichst automatisch, möglichst rasch und unbemerkt vom Anwender ablaufen muss. Die Untersuchungsdauer sollte nämlich idealer Weise nicht wesentlich länger als für ein konventionelles EKG betragen. Dafür sind einige technische Voraussetzungen günstig: Damit möglichst wenige Umschaltungen des eingeleiteten Stromes erfolgen müssen, ist es vorteilhaft den Strom möglichst so einzuleiten, dass die unterschiedlichen Segmente immer mit derselben Stromeinleitung vermessen werden können. Die möglichst konstante Einbringung des Stromes hat den Vorteil, dass sich nicht immer wieder neue Stromfelder aufbauen müssen, wie dies bei unterschiedlicher Einspeisung der Fall ist. Z.B. eignet sich dazu eine Einleitung nahe des oberen Körperendes, z.B. Kopf, Hals oder Nackenbereich einerseits und andererseits das untere Ende der Beine andererseits. So können der Thorax, eventuell auch einzelne Thoraxsegmente, der Rumpf und die Beine sogar gleichzeitig vermessen werden. In vielen Fällen wird auf eine separate Messung der Arme überhaupt verzichtet werden können, da diese nur ca. 7% des Körpervolumens ausmachen und daher die Körpervolumina auf die Einbeziehung der Arme hochgerechnet werden kann. Sollte der Fehler dabei z.B. 10 % ausmachen, würde dies das Gesamtergebnis immer noch um nur 0,7 % verfälschen. Andererseits kann man die Arme deswegen leicht zusätzlich untersuchen, da ja ohnehin beim EKG auch Armelektroden angebracht werden. Es ist natürlich auch daran gedacht, alternativ in jedes Segment separat den Strom einzuspeisen und die Spannung zu messen. Um den Rumpf in zwei Abschnitten und die Beine gemeinsam und/oder separat vermessen zu können, kann die Vorrichtung zur Spannungsmessung entweder mehrfach ausgeführt sein oder aber dieselbe Spannungsmessvorrichtung für die Messung mehrerer Körpersegmente verwendet werden. Da heute innerhalb von Millisekunden ein Messwert ermittelt werden kann und die Abtastung des Impedanzsignals nicht so zeitkritisch ist und daher nicht mit so hoher zeitlicher Auflösung erfolgen muss, kann also zwischen den einzelnen Körpersegmenten hin- und her- geschalten werden. So kann ohne eine Änderung der Stromeinspeisung das Thoraxsegment in Längsrichtung und eventuell auch diagonal in Längsrichtung, das Abdomensegment in Längsrichtung und eventuell auch längsdiagonal und die beiden Beinsegmente gemeinsam und/oder einzeln gleichzeitig gemessen werden.

Zusätzlich kann es von Vorteil sein, von den Impedanzkurven Templates aus den einzelnen Herzschlägen zu erstellen und deren Veränderung mit zusätzlich registrierten Herzaktionen zu erfassen um so den Zeitpunkt zu erfassen, wenn keine wesentliche Änderung der Impedanzkurve mehr eintritt. Zu diesem Zeitpunkt, vielleicht eventuell schon nach ca. 5 bis 20 Herzschlägen kann diese Messung der Impedanz schon abgebrochen werden. Dieses Template kann dann genau hinsichtlich Anstiegssteilheiten Maxima, Minima, Teilflächen und Abfallssteilheiten analysiert werden. Die Erstellung von Templates kann auch für einzelne Phasen des Kreislaufs, z.B. für Inspiration und Exspiration separat erfolgen, weil so z.B. auch die Fluid-Responsiveness und andere Parameter getestet werden könnten. Auch eine Selektion der Templates nach anderen Kriterien, wie z.B. minimale und maximale Herzfrequenz kann hilfreich sein.

Anschließend oder gleichzeitig können in der aus der Anmeldung PCT/AT03/00302 bekannten Weise auch die im wesentlichen selben Körpersegmente ein weiteres Mal mit etwas veränderter Messlänge vermessen werden, um ein gemeinsames Maß für Länge/Fläche des entsprechenden Segmentes zu erhalten.

Anschließend kann auf eine Stromeinspeisung quer zur Längsrichtung übergegangen werden, um die Körpersegmente auch quer zu vermessen. Da diese Quereinspeisung in erster Linie zur Volumenbestimmung des vermessenen Körpersegmentes dient, kann sich die Gewinnung eines Template erübrigen und nur die Grundimpedanz ermittelt werden, so dass die Quervermessung innerhalb weniger Sekunden beendet sein kann. Bei Verwendung der Quermessung zur Erfassung von Inspiration und Exspiration kann auch die Messung länger dauern, wenn man vorteilhafter Weise ein Template während der Inspiration und ein weiteres Template während der Exspiration ermitteln will. So kann dann z.B. auch die Fluid-responsiveness, wie oben beschrieben, erkannt werden. Der gesamte Messvorgang kann daher insgesamt innerhalb einer Minutenfrist beendet sein und dauert daher nicht länger als die eventuell gleichzeitig vorgenommene Registrierung des EKGs. Bei der Verbindung aller am Körper angebrachten Elektroden zum Messgerät ("Function&Spaces-ECG= *FS*-ECG") ist selbstverständlich in bekannter Weise auf einen entsprechenden Schutz des Patienten vor Überspannung und zu großen Stromstärken zu achten, wie dies bei allen Geräten, die in elektrischem Kontakt mit den Patienten stehen, üblich ist. Speziell sollte das Gerät selbstverständlich auch so ausgeführt sein, dass eine Fehlfunktion des Gerätes oder eine Defibrillation des Patienten z.B. bei Kammerflimmern oder Vorhofflimmern trotz angeschlossenem FS -EKG keinen Schaden bei Patienten, Untersuchern oder auch am Gerät auslöst. Dies ist ohnehin Stand der Technik und in allen Geräten, die in der Medizin im Einsatz sind, auf verschiedenste Art und Weise verwirklicht.

Das Gerät und die Vorteile der Methode werden in der Folge in Abb. 1 bis Abb. 18 beschrieben und zwar zeigt
Abb. 1: Ein Zylinderphantom mit stark variiertem Flüssigkeitsgehalt
Abb. 2 Schätzung des Volumens im Phantom durch Impedanz- Vermessung mittels einer Frequenz
Abb. 3: die Vermessung des Phantoms bei mehreren Frequenzen
Abb. 4 die Vermessung des Phantoms bei mehreren Frequenzen und variabler Länge
Abb. 5: die Vermessung des Phantoms bei mehreren Frequenzen und Längs- und Quervermessung
Abb. 6 die Vermessung des Phantoms bei mehreren Frequenzen längs und quer unter Berücksichtigung einer unpräzis vermessenen äußeren Länge und
Abb.7 die Vermessung des Phantoms bei mehreren Frequenzen längs und quer unter Berücksichtigung von präzis vermessener äußerer Länge.
Abb. 8: zeigt das *FS*-ECG von der Seite
Abb.9: zeigt das *FS*-ECG dreidimensional
Abb. 10 zeigt die bevorzugten Elektrodenpositionen und Beschaltungen
Abb. 11 zeigt eine Klemmelektrode mit eingebauter Doppelelektrode
Abb. 12 zeigt den Körper mit aufgebrachten "Spot" Elektroden
Abb. 13 den Körper mit aufgebrachter Kombination von Klemm- und Spot-elektroden
Abb. 14 zeigt den Gerätarm mit Seitenstützen
Abb. 15 zeigt das Impedanzsignal in zwei nachgeschalteten Körpersegmenten
Abb. 16 zeigt die Impedanzänderung nach Injektion einer Flüssigkeit mit einem Wechselstromwiderstand unterschiedlich zu Blut
Abb. 17 zeigt den Befundausdruck von einem gesunden Menschen
Abb. 18 zeigt den Befundausdruck von einem kranken Menschen.
Abb. 19 zeigt eine weitere Elektrodenanordnung
Abb. 20 zeigt eine Untersuchungsmatte die sich in ihrer Form dem Körper anpasst.

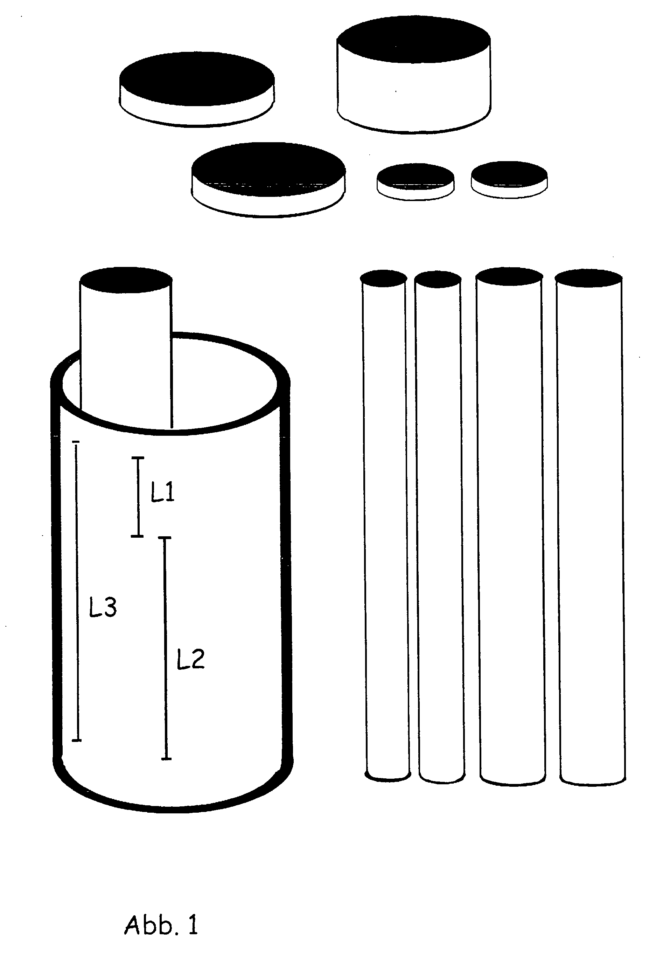

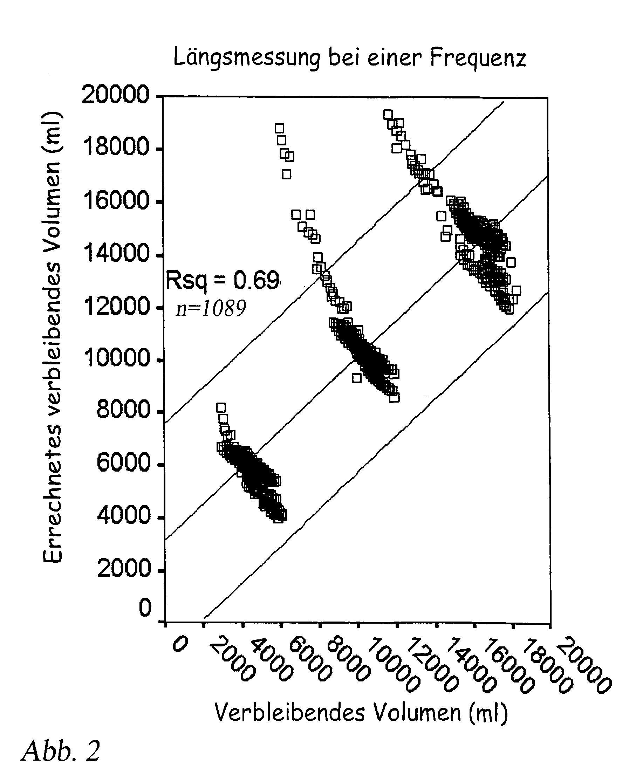

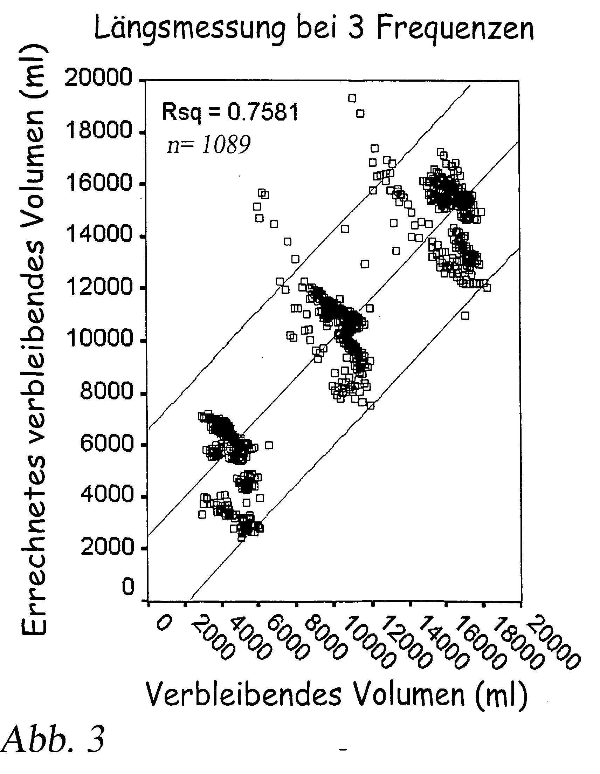

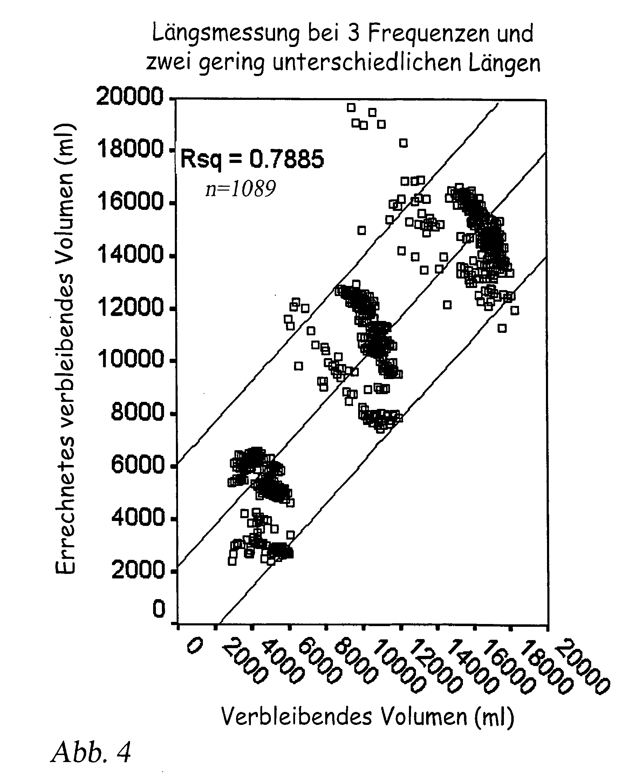

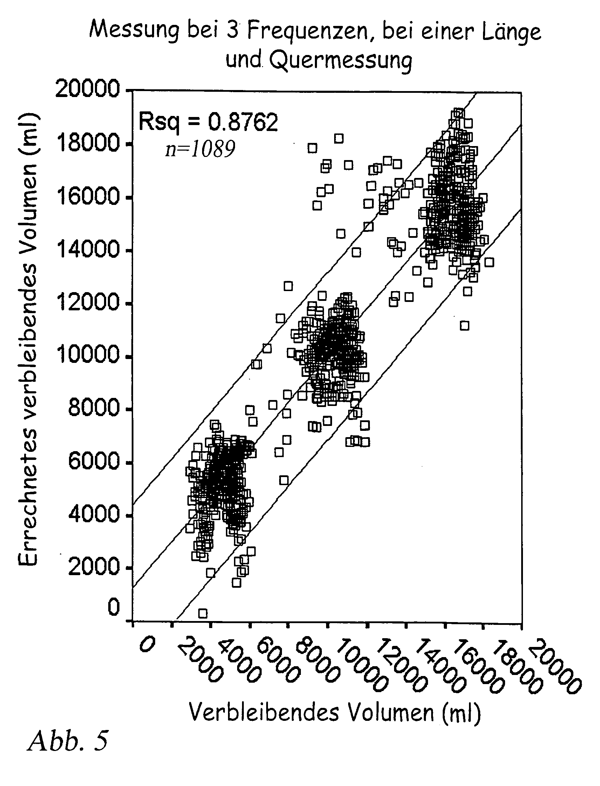

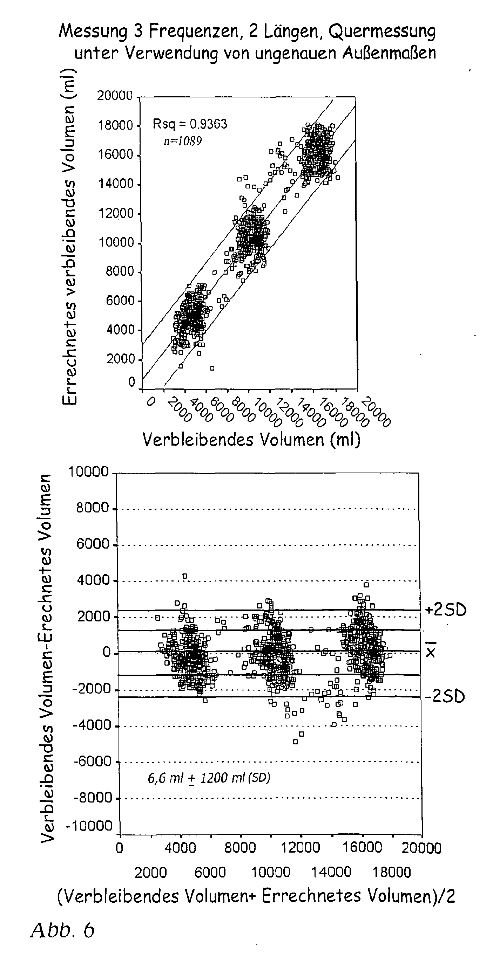

Die folgenden Abbildungen zeigen die Ergebnisse von Untersuchungen an einem Zylinderphantom mit 3 unterschiedlichen Längen in die eine variable Zahl von nicht leitenden Zylindern und Scheiben unterschiedlichen Durchmessers und damit Volumens eingebracht und damit das Flüssigkeitsvolumen innerhalb der drei unterschiedlich langen Zylindersegmente (L1, L2, L3) stark variiert wurde (Abb. 1). Im Anschluss wurde versucht mit Hilfe multipler Regressionsgleichungen das im Zylinder verbliebene bekannte Flüssigkeitsvolumen zu schätzen, wobei Länge und Durchmesser des Zylinderphantoms erst als unbekannt angenommen wurden (Abb. 2 bis 7). Abb. 2 zeigt die Vorhersage des (ja dem Untersucher bekannten) verbleibenden Flüssigkeitsvolumens nur auf Grund der Längsmessung der Impedanz bei einer Frequenz, Abb. 3 eine Schätzung des verbleibenden Flüssigkeitsvolumens auf Grund von Längsmessungen bei einer Distanz und bei 3 Frequenzen, Abb. 4 zeigt eine Schätzung des verbleibenden Flüssigkeitsvolumens auf Grund von Mehrfrequenzmessungen und der Längsmessung bei zwei gering unterschiedlichen Längen, wie in der Patentanmeldung PCT/AT 03/00302 beschrieben. Abb.5 die Volumenschätzung auf Grund von Mehrfrequenzmessungen bei jeweils *einer* Längs- und Quermessung der Impedanz. Abb. 6 zeigt die Schätzung des verbleibenden Volumens auf Grund von zwei Längs- und einer Quermessung der Impedanz bei mehreren Frequenzen, zusätzlich wurde die Länge des Segmentes auf ± 2cm genau eingegeben. Die ± 2 cm wurden als optimistische Annahme der in-vivo erzielbaren Genauigkeit gewählt. Dafür wurde mittels eines Zufallsgenerators die wahren Maße des Zylinders um die angegebenen ± 2cm , eine in der Praxis kaum zu erreichende Dimension variiert. Selbst die schlechte Präzision von ± 2 cm ist in der Praxis durch die Komplexität des menschlichen Körpers nicht zu erzielen, deshalb handelt es sich nicht nur um realistische, sondern um eine äußerst optimistische Annahme.. Wie aus den Abbildungen 2 bis 6 ersichtlich, nimmt die Vorhersagemöglichkeit des verbliebenen "unbekannten" Flüssigkeitsvolumens beträchtlich zu, wenn der Zylinder mit seiner extrem inhomogenen Flüssigkeitsverteilung bei mehreren Frequenzen, bei zwei unterschiedlichen Längen oder auch längs und quer vermessen wurde.

Wie ersichtlich ist dabei die Quermessung der Impedanz (Abb.5) bei der Schätzung des Volumens die Messung der Impedanz bei zwei unterschiedlichen Längen (Abb. 4) deutlich überlegen. Die Nützlichkeit der Messung des Phantoms bei mehreren Frequenzen ist erstaunlich, da ja durch die Befüllung des Zylinders mit einem Elektrolyten nur ein Kompartment vorhanden war. Dies hängt sicher unter anderem mit der dadurch besseren Beurteilung der Inhomogenitäten zusammen. Dies unterstreicht die Bedeutung der Messung jedes Kompartments bei zumindest zwei für dieses Kompartment charakteristischen jedoch möglichst unterschiedlichen Frequenzen. Noch günstiger kann natürlich ein kompletter Frequenz-Sweep über das komplette Frequenzband z.B. von 1 kHz bis 2 MHz oder Teile davon sein. Wie ersichtlich beträgt die Abweichung des geschätzten vom tatsächlich vorhandenen Flüssigkeitsvolumens in Abb. 6 noch immer ± 2400 ml !!(± 2SD). Es muss angemerkt werden, dass trotz der Verbesserung der Vorhersage durch Längs- und Quermessungen die Genauigkeit der Methode absolut unzureichend ist, so täuscht das Bestimmtheitsmaß von 0.94 darüber hinweg, dass das Volumen etwa nur halb so groß oder auch doppelt so groß wie geschätzt sein kann, wie die Abb. 6 zeigt. Dies macht die Methode für die Klinik unbrauchbar. Beim Menschen wird die Situation ja auch zusätzlich noch wesentlich komplexer, weil parallel geschaltete und seriell geschaltene Resistance and Reactance nebeneinander existieren, während in dem einfachen gezeigten in vitro Modell der Zylinder ja nur mit einem Elektrolyten aber nicht mit lebenden Zellen mit ihren als Dipol wirkenden Zellmembranen gefüllt war.

Abb. 7 hingegen zeigt den drastischen Informationssprung, um wie viel besser, die Schätzung des verbliebenen Flüssigkeitsvolumens nochmals wird, wenn die äußeren Dimensionen des Zylinders mit Querschnitt und Länge, das "containing volume", nämlich auf einen Millimeter genau bekannt sind, und in die Vorhersagegleichung mit einbezogen wird. Bekanntlich kann mit Hilfe der beschriebenen volumetrischen Verfahren die Körperoberfläche mit einer Genauigkeit von weniger als 1mm Abweichung vermessen werden. Wie aus Abb. 7 ersichtlich, wird jetzt plötzlich die Methode ausreichend genau für die klinische Anwendung, nämlich beträgt der Fehler jetzt plötzlich nur mehr 1058 ml (± 2SD) des Messwertes, wobei nur in diesem Fall die bemerkenswerte Mehrzahl der Werte innerhalb von nur ± 529 ml liegen und nur einzelne Ausreißer die hohen 2 SD bewirken. Die verschiedenen Längs- und Quermessungen der Impedanz tragen jedoch auch bei genauer Kenntnis der äußeren Dimensionen der wahren Länge des Zylinders noch immer hochsignifikant zur Vorhersage bei, wie die folgende Tabelle zeigt:

| Modell | T-Wert | Siginifikanz (p<) |
|---|---|---|
| Konstante | 3,4: | 0,001 |
| Längsimpedanz 5 kHz | -4,3 | 0,000 |
| Längsimpedanz 40 kHz | -9,0 | 0,000 |
| Längsimpedanz 400 kHz | +7,5 | 0,000 |
| Längsimpedanz kurz 400 kHz | -7,3 | 0,000 |
| Impedanz Quer 40 kHz | -6,3 | 0,000 |
| Impedanz Quer 400 kHz | +6,3 | 0,000 |
| Genaue äußere Länge | +93,3 | 0,000 |

Dabei ist noch nicht berücksichtigt, dass natürlich bei Kenntnis der wahren äußeren Querschnittsfläche des Leiters (des menschlichen Körpers) eine noch bessere Definition der Geometrie und damit des Flüssigkeitsanteiles in diesem Körper möglich ist. Da der Querschnitt der menschlichen Körperteile mit ihren Konturunregelmäßigkeiten, Verformungen und Ausbuchtungen nicht wirklich mit dem Maßband messbar ist, wird die hier vorgestellte Volumetrie einen zusätzlichen weiteren bedeutenden Fortschritt bei der elektrischen Messung von Körperräumen (Spaces) darstellen. Die vorgestellten Ergebnisse unterstreichen die Wichtigkeit der kontaktlosen Messung des Absolutvolumens des Körpers wie in dieser Schrift ausgeführt.. So wird in Hinkunft nicht nur das Absolutvolumen des Körpers sehr genau sondern, auch viel genauer als bisher die Volumsanteile von Körperwasser und Fett zu ermitteln sein. Bei Kenntnis des spezifischen Gewichts von "fat free mass" (= circa 1,1) und fat mass (= circa 0,9) kann dann auch ohne Wägung des Patienten das Gesamtkörpergewicht estimiert werden.

Die folgenden Abbildungen zeigen die verschiedenen Ausprägungen des *FS-*ECG:
Abb. 8 zeigt beispielhaft einen Gerätearm -1- , der z.B. zumindest vorübergehend in konstanter Position zur Untersuchungsliege -2- (siehe Abb. 2) ist oder auch an der dieser befestigt sein kann. In diesem Gerätearm -1- könnten auch berührungslose Messgeräte -3- für physikalische Größen untergebracht sein. Eine wichtige Größe ist z.B. das Volumen des untersuchten Körpers -4-. Es werden ja im Rahmen des *FS-*EKGs die Flüssigkeitsmengen, z.B. das intrazelluläre und das extrazelluläre Volumen ermittelt. Diese absoluten Volumina, nämlich zB. das Extrazelluläre und Intrazelluläre Wasser, Beinödeme, Ascites, Lungenödem usw. können dann nicht nur genauer errechnet werden (siehe Abb. 7), sondern können dann in der Folge auch in echten Litern und in % des Körpervolumens angegeben werden, wie das z.B. für ECF, ICF, TBW der am besten etablierte Standard ist. Bei Kenntnis des Volumens des untersuchten Körpers bzw. seines Wasseranteiles (und damit auch des Nichtwasser- nämlich Fettanteiles) kann dann auch sehr genau aus dem spezifischen Gewicht von Wasser und Fett das Gesamtgewicht des untersuchten Körpers errechnet werden. Dies ersetzt auch das Abwiegen von Patienten was bei Schwerkranken faktisch unmöglich ist, da sie weder auf eine Steh- und in vielen Fällen nicht auf eine Sitzwaage befördert werden können.

Mit -3-, sind daher ein oder eventuell mehrere berührungslose Messgeräte -3-für physikalische Eigenschaften gezeigt, mit dem z.B. auch das Volumen des untersuchten Körpers -4.- und auch die Position der aufgebrachten Elektroden -5a, 5b, 5c, 5d- bzw. der dazugehörigen Elektrodenhalterungen -6a,6b, 6c, 6d- erfasst werden können. Dabei könnte es sich z.B. um Distanzmessgeräte und eventuell zusätzlich Winkelmessgeräte handeln, wie sie aus der Technik bereits gut bekannt und wie sie kostengünstig serienfertig zu kaufen sind. Dabei könnte es sich z.B. um Radio- Ultraschall- oder optische Messgeräte handeln, die in bekannter Weise die physikalischen Eigenschaften des Körpers, wie z.B. das Volumen (das sich aus der Distanz der Körperteile im Verhältnis zur Distanz der Untersuchungsliege ergibt) ermitteln lassen. Üblicherweise wird dafür entweder eine Interferometry, "Time-of-Flight" oder eine Triangulations- Methode verwendet. So könnte das reflektierte oder gestreute Signal analysiert werden. Es könnte im einfachsten Fall jedoch auch nur ein digitales Abbild des Körpers und/oder seiner Abschnitte z.B. mittels CCD oder CMOS in mehr als einer Ebene angefertigt werden, um damit mit Hilfe spezieller Algorithmen das Volumen des Körpers und seiner Segmente zu errechnen. Gleichzeitig können so auch die Distanzen zwischen den einzelnen Elektroden voneinander ermitteln werden. Dazu müsste nur durch spezielle unverwechselbare Formgebung jeder einzelnen interessierenden Elektrode (siehe z.B. -5a-, -5b-, -5c-, -5d-) von dem/den berührungslosen Messgeräten -3-erkannt werden. Bei Kenntnis der Distanz und des Winkels von den einzelnen Elektroden zum Distanzhalter kann sofort die Distanz zwischen den Elektroden ermittelt werden. Durch eine charakteristische Profilgebung der Elektroden- oder deren Elektrodenhalterungen (6a-,-6b-,-6c-, -6d-) kann auch sofort die richtige Applikation der Elektroden erkannt werden. Die wahre Distanz zwischen den Elektroden ist deswegen wichtig zu erkennen, weil bei Kenntnis der wahren Distanz eine etwaige Fehlhydrierung in diesem Körpersegment noch besser erkannt werden kann. Dies ist deswegen so entscheidend, da bei wiederholter Untersuchung des Körpers eine Fehlposition der Elektrode 5a, 5b, 5c, 5d um nur ±2 cm bei einem Segmentdurchmesser von ca. 18cm (z.B. Oberschenkel) bereits einen Unterschied von 1 Liter Flüssigkeit ausmachen würde. Bei einem Thoraxdurchmesser von ca. 30 cm würde dies sogar 2,8 Liter ausmachen. Damit wäre bereits die Methode unbrauchbar. Bei der berührungslosen Oberflächenerfassung liegt die Genauigkeit im Bereich von 1mm (z.B. (Sick Ranger oder LMS 400 Laser) was beim angeführten Beispiel einem Fehler von 0,070 Liter statt 2,8 Litern entspricht.. Besonders vorteilhaft zur Entdeckung der Elektrodenposition sind dabei Elektroden-5 a, 5b, 5c, 5d- oder Elektrodenhalterungen -6a,-6b, -6c, -6d- die auf Grund von speziellen physikalischen Eigenschaften wie in Abb. 8 angedeutet (z.B.. Profilgebung, Farbe, einer speziellen Reflexion, Schwingung, Temperatur usw.) als eindeutiges Ziel des Oberflächenerfassungsgerätes identifiziert werden können. Damit könnte dann auch sofort eine fehlerhafte Anbringung oder Vertauschung der Elektroden erkannt und dem Benutzer signalisiert werden, wenn die einzelnen Elektroden eine jeweils spezielle Kennung aufweisen. So könnte mit dem/den berührungslosen Messgerät -3- der auf der Untersuchungsliege -2- liegende Patient genau dreidimensional vermessen werden. Durch das dreidimensionale Abbild des Patienten können dann sehr einfach die Volumina der einzelnen Segmente ermittelt werden und die Fehlhydrierung und der Fettanteil in den einzelnen Kompartments ermittelt werden. Vorteilshafterweise sind die Elektroden nicht ganz in der Körperperipherie (sprich Hände und Füße, sondern zentraler am distalen Unterschenkel und am distalen Unterarm) angebracht, um nicht einen unnötigen Vorwiderstand in den sich verjüngenden Extremitäten hervorzurufen. Um trotzdem dann in der Folge das gesamte Volumen der Kompartmente zu erfassen, bewährt es sich sehr mittels der berührungslosen Volumenmessung auf den Rest des Körpers außerhalb der Elektroden zu extrapolieren. Um den ganzen Körper bezüglich Distanz und anderer physikalischen Größen abzuscannen könnte man z.B. das berührungslose Messgerät -3-, entweder nur mittels Schwenkantrieb -7- schwenken., wobei der Winkelmesser und der Entfernungsmesser ebenso ein wirklichkeitsgetreues Abbild des Körpers erzeugen können, da ja die Untersuchungsliege -2- , auf welcher der untersuchte Körper -4- liegt durch ihre konstante (meist flache) Formgebung als Referenz herangezogen werden kann, so dass die eventuell entstehende Verzerrung des untersuchten Körpers leicht mathematisch ausgeglichen werden kann. Bei Schwenken der berührungslosen Messgeräte mittels Schwenkantrieb -7- ist zu beachten, dass die Messfelder-8- von mehreren berührungslosen Messgeräten -3- sich so überlappen, dass eine komplette dreidimensionale Abbildung des Körpers möglich ist. Die Elektroden -5a bis 5d- bzw. die Elektrodenhalterungen 6a-bis 6d-sind über Kabel -9- mit dem FS-EKG -10- verbunden, wobei in dieser Abbildung nur die Kabel von einer Körperseite, nicht jedoch von der gegenüberliegenden Körperseite eingezeichnet sind. Die Untersuchungsliege -2- als Referenzfläche ist deswegen so wichtig, weil üblicherweise der Körper von allen Seiten abgebildet werden muss, um das tatsächliche Volumen zu ermitteln. Dies ist bei schwerkranken Patienten nicht möglich, daher muss die Untersuchungsliege -2- als hintere Referenzfläche angenommen werden. Dies ist besonders gut möglich, wenn die Fläche relativ hart ausgebildet ist, weil sie sich dann nicht weiter verformen kann. Damit kann die Hinterfläche des nicht abgebildeten Körpers als relativ eben angenommen werden. Aber auch wenn sie nicht so hart ausgebildet sein sollte, wird ja im Grenzbereich zwischen Körper und Untersuchungsliege -2- dreidimensional erfasst, inwieweit sich die Liege unter dem Gewicht des Körpers verkrümmt, so dass trotzdem genau das Volumen des Körpers errechnet werden kann. Speziell könnte der elastische Modulus der Liege bekannt sein, der in die Berechnung des Volumens des Körpers -4- eingeht. Andererseits könnte das berührungslose Messgerät -3- auch mittels -Schiebeantrieb -11-entlang des Gerätearms -1- verschoben werden. Der Schiebeantrieb -11- könnte als x und/oder x/y Antrieb ausgeführt sein, um das berührungslose Messgerät -3- mit konstantem Messwinkel -12- über den Körper -4- zu bewegen, um so ein genaues dreidimensionales Bild vom untersuchten Körper und seinen Segmenten zu erhalten. Der Schiebeantrieb -11-ist dabei nur für eines der beiden eingezeichneten berührungslosen Messgeräte -3- gezeigt, müsste natürlich jedoch für alle vorhanden sein, sofern nicht ein unbewegtes berührungsloses Messgerät -3- ohne Schwenk oder Verschiebung ein komplettes 3-D Bild des Körpers erzeugt. Bei der verwendeten Methode ist zu beachten, dass bei der Verwendung von Ultraschall üblicherweise eine Kegelförmige Ausstrahlung erzeugt wird, während sich bei optischen, Laser und Radiosignalen das Signalbündel sehr gut fokussieren lässt. Damit wird wahrscheinlich den optischen Methoden, eventuell auch im unsichtbaren Bereich, eventuell im Infrarotbereich, der Vorzug zu geben sein.

Bei Ausstattung der entsprechenden berührungslosen Messgeräte -3- mit anderen entsprechenden Sensoren, können auch andere physikalische Unterschiede wie z.B. auch Temperaturunterschiede zwischen einzelnen Körperteilen (Thermographie) somit sofort erkannt und für die Diagnose verwendet werden. Als Beispiele werden nur genannt Erwärmung durch lokale Entzündungen oder auch Unterkühlung durch lokale Minderdurchblutung. Außerdem sei beispielsweise genannt das Verfahren der aktiven Thermographie, bei dem die Temperaturableitung durch das umliegende Gewebe untersucht wird. Auch dies lässt Flüssigkeitsansammlungen im darunter liegenden Gewebe, wie z.B. durch Hydrops oder Erguss bedingt leichter erkennen.

Die Untersuchungsliege -2- weist vorteilhafter Weise, wie gezeigt, eine über die Horizontale ausgerichtete Liegenoberteil -13- auf, da ja oft auch herzkranke Patienten untersucht werden sollen, die oft nicht flach gelagert werden können. Der Winkel für die Neigung sollte vorteilhafter Weise zwischen 20 und 45 Grad, z.B. auch 30 Grad betragen. Eine automatische oder händische Vermittlung des Neigungswinkels -14- des Liegenoberteils -10- sollte vorgesehen sein. Auch eine unter die Horizontale ausgerichtete Neigung für die untere Körperhälfte kann von Vorteil sein. Bei fixer Neigung ist auch die Messung der beschriebenen physikalischen Größen zum Patienten leichter zu ermitteln. Auch zum Vergleich der Messwerte des selben Patienten zu verschiedenen Zeitpunkten sollte dieser unter gleichen Neigungswinkeln untersucht werden, da es sonst zu Flüssigkeitsverschiebungen kommt, welche die Messwerte verfälschen.

Auch könnte die Untersuchungsliege physikalische Größen vermitteln, die mit Hilfe der oben beschriebenen kontaktlosen Methoden nicht erfasst werden können. So könnten z.B. unter dem Patienten, in die Liege eingebaut, oder in einer Matte angebracht, einer oder mehrere Druck-sensible Sensoren eingebaut sein, die das Gewicht des Patienten bzw. seiner Körpersegmente erfassen lassen. (nicht gezeigt).

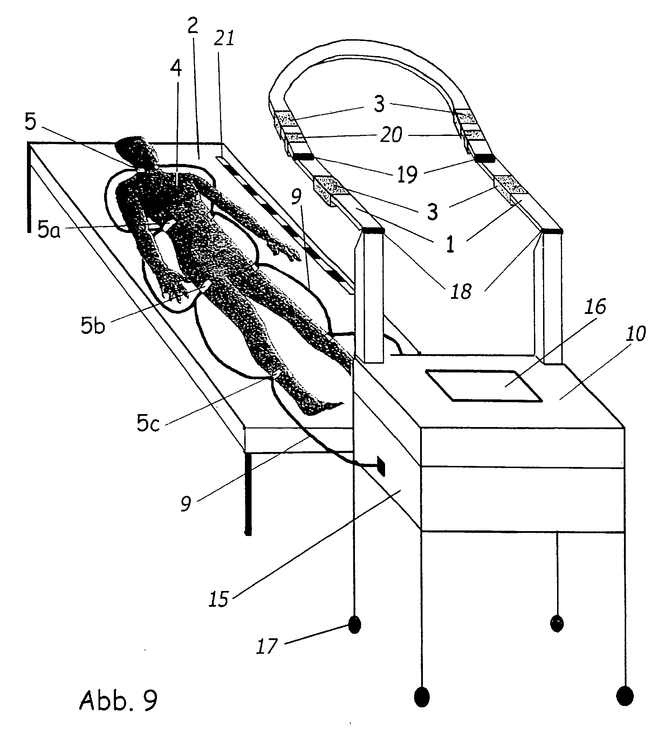

Wie in Abb. 9 gezeigt, könnte man den Gerätearm -1- auch an einem Messwagen -15- montieren. Dies ist deswegen günstiger, weil mit der Positionierung des FS-EKG -10- mit dem Anzeige-Monitor -16- am Messwagen -15- dieser sehr leicht mit den vorhandenen Rollen -17- von Patient zu Patient befördert werden kann. Für den Transport ist es wichtig dass der Gerätearm -1- mit Hilfe einer Schwenkvorrichtung -18- in eine senkrechte Position am besten längs des Messwagens -15- gebracht werden kann. Der Gerätearm -1- könnte mit Zusatzgelenken -19- (z.B. Teleskopmechanismus, Knickverkürzung usw.)ausgestattet sein, die diesen so verkürzen, dass er sich beim Transport des Messwagens nicht sperrig wegsteht sondern sich in Flucht mit dem Messwagen -15- befindet. Die Kabel -9- vom FS-EKG -10- zum Körper -4- sind beispielsweise als Bündelkabel ausgeführt, damit die 3-D- Erfassung des Körpers -4-möglichst wenig gestört wird.

In der Praxis wird es genügen, das/die berührungslosen Messgeräte -3- weder zu schwenken noch zu verschieben, sondern den Körper mit mehreren Messgeräten gleichzeitig dreidimensional zu erfassen, wie das in Abb. 9 dargestellt ist. In dieser Abbildung ist der Gerätearm -1-, doppelt ausgeführt, um so besser ein dreidimensionales Bild vom Körper gewinnen zu können. Die berührungslosen 2D oder 3D Messgeräte -3- sind beispielsweise im U-förmigen Gerätearm -1- jeweils doppelt ausgeführt. Damit lässt sich sehr rasch ein komplettes 3-D Bild des Körpers ermitteln, wobei durch die seitliche Positionierung zum Körper -4- gewährleistet ist, dass die kritischen Randbereiche zwischen Körper -4- und Untersuchungsliege -2- besonders genau dargestellt werden können, um z.B. auch eine eventuelle Verformung der Untersuchungsliege -2- erkennen. zu können. Die Ausstattung mit z.B. einfachen und billigen CCDs oder CMOS als berührungslose Messgeräte -3- oder ähnlichem und unter Verwendung eines Gitter- Projektors -20- z.B. eines Infrarot-Gitters oder auch Punkt- oder Linienprojektors, könnte in Sekunden unbemerkt die Berechnung des Körpervolumens und seiner Segmente ermöglichen. Dies wäre deswegen besonders wichtig, da Bewegungsartefakte des Körpers -4- völlig ausgeschaltet werden. Ein gleichzeitig mit abgebildeter Messstab -21- lässt jederzeit den unterschiedlichen Abstand zwischen Gerätearm -1- und Untersuchungsliege -2- berücksichtigen: Andererseits kann der U-förmige Gerätearm -1- dazu dienen, z.B. mittels des Schiebeantriebes -11- (siehe Abb. 8) bzw. auch über Schwenkung ein z.B. einzelnes berührungsloses Messgerät -3- dazu zu nützen, um den Körper -4- aus mehreren Positionen zu erfassen, was allerdings den Nachteil des zeitlichen Abstandes der Messungen hat.

Sollte die 3-D Vermessung des Körpers dazu dienen, um gleichzeitig mit Hilfe der Impedanz das Extrazellulärvolumen und Intrazellulärvolumen im Körper zu erfassen, verlangt die Vermessung der jeweils annähernd zylindrischen Körpersegmente sowohl im Durchmesser als in ihrer Länge eine spezielle Elektrodenanordnung, die im folgenden an Hand der Abbildungen näher beschrieben wird.

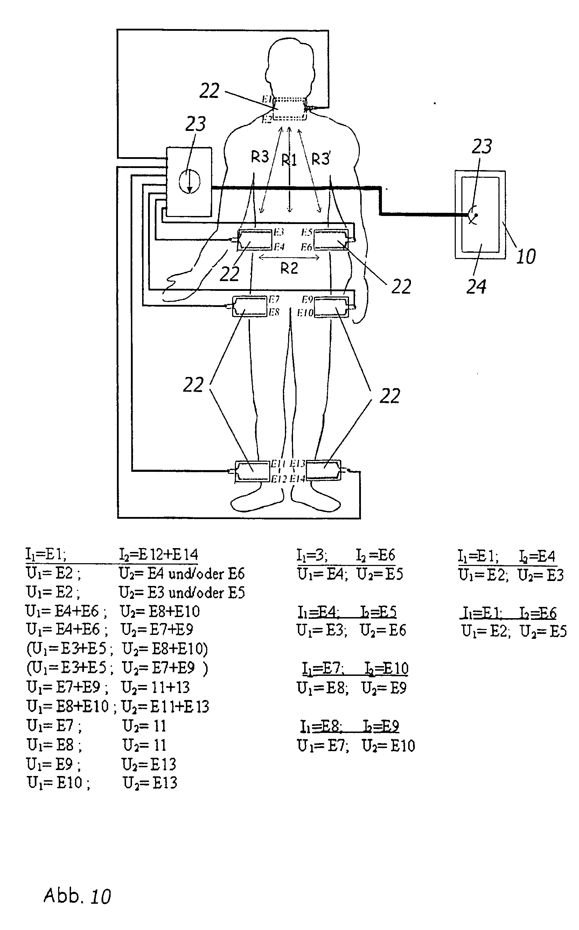

Abb. 10 zeigt mögliche Anbringung von Elektroden für die Stromeinleitung und Spannungsmessungen geeignet zur genauen Erfassung der Körpervolumina, der Herzaktion und der Durchblutung in den verschiedenen Körperabschnitten. Mit -22- sind dabei die verwendeten Doppelektroden gekennzeichnet, die hier z.B. bandförmig ausgeführt sind. Genauso gut könnten sie jedoch eine x-beliebige Form aufweisen , so z.B. auch als "Spot-Elektroden" ausgeführt sein. Mit -23- ist der Messstellen- und Stromeinspeisungs-Umschalter (kurz: Umschalter) erkenntlich, mit dessen Hilfe sichergestellt wird, dass jeweils richtige Segment entweder quer, der Länge nach, oder auch diagonal, (bei Wegschalten der einen jeweiligen Elektrode in gleicher Körperhöhe) vermessen wird und mit dem gegebenenfalls auch die Stromeinspeisung umgeschaltet werden könnte. Mit -10-ist das FS-ECG eingezeichnet, das auch eine Konstantstromquelle und eine Impedanzmessgerät -24- beinhaltet. Dies könnte ein Mehrfrequenzimpedanzgerät inklusive Wechselstromquelle enthalten. Dieses kann z.B. diskrete Frequenzen z.B. 5, 40, 200 und 400 kHz oder auch einen kompletten Frequenz-Sweep über jeden gewünschten Messbereich erzeugen und messen. Die bevorzugten Elektrodenpositionen werden mit E1 bis E 14 bezeichnet., . E 1 und E2 befinden sich dabei am Nacken, E3 bis E6 jeweils beidseits am Thorax" E7 bis E10 jeweils beidseits am Beinansatz und E11 bis E14 jeweils beidseits an den distalen Unterschenkeln. Es wäre günstig, alle Elektroden ähnlich auszuführen, um die Bedienung zu vereinfachen. So könnten alle Elektroden, nämlich EKG und Impedanz Elektroden als Klebeelektroden ausgeführt sein. Für die Kurzzeitmessung könnte es sich als günstig erweisen nur einen schwach haftenden Klebstoff zu verwenden, der sich sehr leicht und schmerzlos wieder vom Körper läsen lässt, ähnlich wie er z.B. von den "post it" Aufklebern bekannt ist. Andererseits könnten alle Elektroden als Saugelektroden ausgeführt sein, wie das vom EKG her bekannt und üblich ist. Für Langzeit- oder Dauermessungen hingegen werden gut klebende Elektroden günstig sein.

Im unteren Teil der Abb. 10 sind die vorteilhaftesten Kombinationen der Strom- und Spannungsmessung- Umschaltung angeführt, die natürlich bei Bedarf ergänzt werden können. Für die Quermessung wird vorteilhafter Weise jeweils die eine Elektrode der Doppelelektrode als Strom- die andere als Spannungselektrode beschaltet. Da eine echte Vierpunktmessung, mit außen liegenden Strom- und innen liegenden Spannungselektroden quer am Rumpf, Abdomen und den Beinen schlecht möglich ist, wird vorgeschlagen, jedes der auf gleicher Körperhöhe liegenden Doppelelektrodenpaare diagonal und alternativ als Strom- und Spannungs-Elektrode zu beschalten, wie das im rechten unteren Teil der Abb. 10 gezeigt ist. Der Mittelwert der beiden jeweiligen Diagonalmessungen ist ein sehr gutes Maß für die Querimpedanz und damit für den Flüssigkeitsquerschnitt des entsprechenden Körpersegmentes. Weiters ist auch gezeigt, dass es vorteilhaft sein kann mittels der Elektroden zwischen den Elektrodenpositionen E1 einerseits und E12 und E14 andererseits den Strom einzuleiten und andererseits a) zwischen den Elektrodenpositionen E2 und E3, b) zwischen E2 und E4, andererseits zwischen c) E2 und E5 und außerdem zwischen d) E2 und E6 die Spannung zu messen. Dabei wird der Thorax diagonal bei jeweils zwei verschiedenen Thoraxlängen in den Richtungen R3 und R3'zu vermessen um so, auch sofort einen einseitigen oder zweiseitigen Pleuraerguss zu erkennen. Zusätzlich ist auch daran gedacht, anfänglich vor Beginn der Messung die richtige Positionierung der Elektroden und den richtigen Anschluss derselben elektrisch zu überprüfen. Dies ist insofern leicht möglich, als bei richtiger Beschaltung der Doppelektroden, die jeweils äußere, vom Zentrum des Körpers entferntere Elektrode einen höheren Wechselstromwiderstand aufweisen muss, als die näher beieinander liegenden Elektroden. So kann auch bei falscher Anbringung die falsche Elektrode im Nachhinein richtig beschaltet werden. Die beschriebene Ausführung ist wesentlich ökonomischer, als mehrere Impedanzmessgeräte zu verwenden was natürlich alternativ auch denkbar wäre.

Mit -24- ist das Mehrfrequenzimpedanzgerät inklusive Wechselstromquelle als Teil des FS-ECG symbolisiert. Dieses kann z.B. diskrete Frequenzen z.B. 5, 40, 200 und 400 kHz oder auch einen kompletten Frequenz-Sweep über jeden gewünschten Messbereich erzeugen und messen.

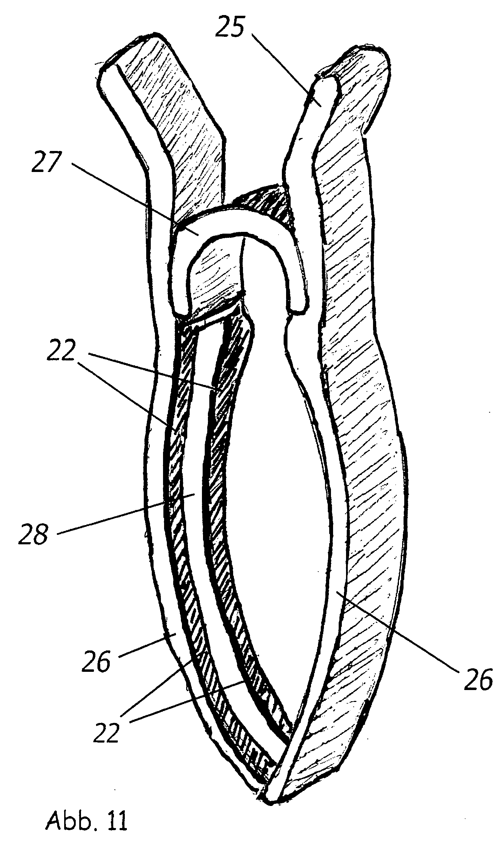

Abb. 11 zeigt eine weitere Elektrodenausführungsform, wobei an den Körperteilen eine elastische Klemme -25- angebracht werden kann, die an den gegenüberliegenden Bügeln
-26- zumindest eine oder auch zwei Doppelelektroden -22-, die durch die Spannvorrichtung -27- der elastischen Klemme -25- guten Kontakt mit dem Körper finden. Die Bügeln -26- sollten dabei so ausgeführt sein, dass eine Isolierung zwischen den Doppelelektroden -22- gewährleistet ist. Dabei könnte z.B. der Bügel nur eine Vertiefung-28-, die nicht am Körper -4- anliegt, zwischen den Doppelelektroden aufweisen.

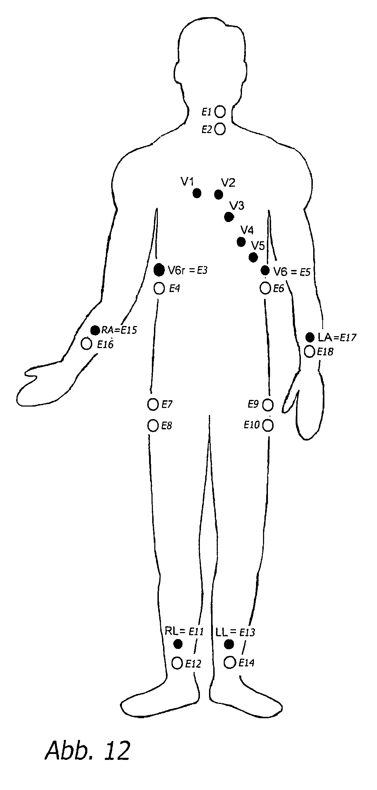

Abb. 12 zeigt den menschlichen Rumpf, bei dem jetzt die selben Elektroden, die in Abb. 10 bandförmig ausgeführt sind, jetzt als Spotelektroden ausgebildet sind. Die Nummerierung entspricht dabei der in Abb. 8, jedoch werden zusätzlich, schwarz ausgeführt, die konventionellen EKG Platzierungen gezeigt, wobei mit RA die Elektrode am rechten Arm, mit LA die Elektrodenplatzierung am linken Arm, mit RL die Elektrodenplatzierung am rechten Bein (right leg=RL), mit LL die Elektrodenplatzierung am linken Bein (left leg=LL) Vorteilsweise werden die Beinelektroden RL, LL bzw. E11 bis E14 am Wadenmuskel und nicht weiter distal angebracht, weil dies nur durch das weiter distal liegende schlecht leitende Gewebe, einen "Vorwiderstand" verursachen würde, der unnötig und unkontrollierbar die Impedanz erhöhen würde. Anschließend kann ja mittels der berührungslosen Volumenmessung der außerhalb der Elektroden liegende Körperteil zu den Körperräumen hochgerechnet und dazuaddiert werden.

Mit V1 bis V6 sind die konventionellen Brustwandelektroden gezeigt. V6 kann dabei als ICG Elektrodenposition E5 (oder E6), RL als ICG Elektrodenpositionen E11 (oder E7,E8, E12,), LL als ICG Elektrodenposition E13 (oder E9,E10,E14) mit verwendet werden. Auch die ICG Elektrodenposition E3 könnte zusätzlich als EKG Elektrodenposition V6r verwendet werden. Diese Elektroden könnten z.B. , wie bekannt, als Saugelektroden in Verbindung mit einer Unterdruckquelle (nicht gezeigt) ausgeführt sein, wie das heute vielfach üblich ist. Dabei könnten z.B. auch Elektroden, die in konstanter Lage zueinander sein müssen, wie die hier gezeigten Elektroden V 6 und E6 auf einem gemeinsamen Träger - 29- befinden, sodass auf alle Fälle der richtige Abstand der Klebe- oder Saugelektroden zueinander garantiert ist. Auch die EKG-Elektrodenpositionen RA und LA könnten verwendet werden, um auch die Armsegmente mittels Impedanz zu vermessen, wobei RA z.B. als zusätzliche IKG Elektrodenposition E15 (oder E16) und LA als zusätzliche IKG Elektrodenposition E 18 (oder E19) verwendet werden könnte. Die Stromeinleitung würde dabei über die Elektrodenposition E16 und E1 einerseits und E18 und E1 andererseits erfolgen, die Spannungsmessung jeweils zwischen E2 und E15 einerseits und zwischen E2 und E17 andererseits. Auch für die anderen Elektrodenpositionen die in konstantem Abstand zueinander sein sollen, könnte ein gemeinsamer Träger vorhanden sein (nicht gezeichnet).

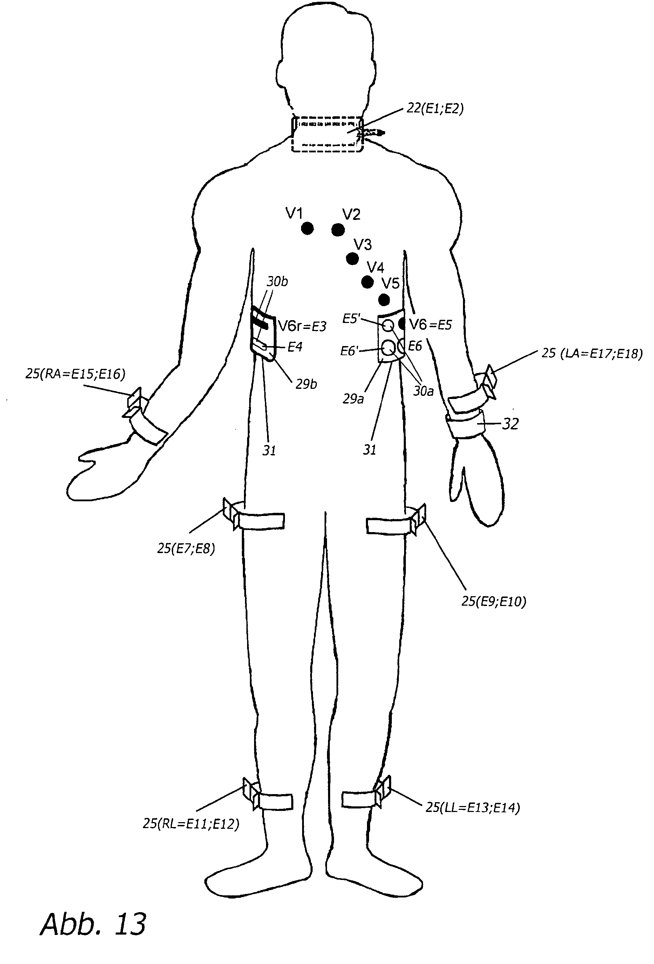

Abb. 13 zeigt die wahrscheinlich in der Praxis vorteilhafteste Ausführungsform der Elektrodenanordnung weil sie sich weitgehend an das konventionelle EKG anlehnt und daher keine Umschulung des Personals erforderlich ist. Dabei werden auch aus Kostengründen in bekannter Weise wieder verwendbare elastische Klemmen -25- angelegt (wobei diese jetzt Doppelektroden enthalten, wie in Abb. 11 gezeigt) und am Brustkorb auch aus ökonomischen Gründen Saugelektroden, die das Personal bereits vom konventionellen EKG gewöhnt ist. Dabei wird auch gezeigt, dass die Doppelektroden (Abb. 10; -22-) am Brustkorb auch als Doppelsaugelektroden -30a- und -30b-auf einem gemeinsamen Träger-29- ausgeführt sein können. Mit 30a ist dabei gezeigt, dass z.B. mehrere, z.B. 4 Spotelektroden gemeinsam auf einem Träger -29- (z.B. Saugelektrode) untergebracht sind, um einen reproduzierbaren Abstand der Elektroden (E5, E6) zu gewährleisten. Wenn die Elektroden E5 und E5' bzw. die Elektroden E6 und E6' miteinander galvanisch verbunden sind, entsteht die Wirkung einer annähernd bandförmigen Elektrode. Andererseits könnte die Saugelektrode auch zwei annähernd bandförmige Elektroden enthalten, wie das mit -29b-bezeichnet ist. Mit -30- ist dabei symbolhaft die Abdichtung zum Körper gekennzeichnet, wobei natürlich auch jede einzelne Elektrode in bekannter Weise als separate Saugvorrichtung ausgeführt sein könnte. Zur Verlaufskontrolle wird man sich in vielen Fällen mit den peripheren EKG Ableitungen begnügen, sodass dann auch die Brustwandelektroden V1 bis V5 wegfallen können. Lediglich die Doppelelektrode -22- im Nacken könnte als Einmalklebeelektrode ausgeführt sein um auch "Disposables" zu generieren. Dazu könnte beispielsweise die Nackenelektrode so gefertigt werden, dass sie sich mit Sicherheit nur für die einmalige bzw. begrenzte Verwendung eignet. Dazu könnten auch z.B. ein Barcode, der eingelesen werden muss, oder elektronische Sicherungen in die Elektrode eingebaut sein, die das FS-EKG sofort erkennen lassen, ob die Elektrode bereits verwendet wurde, bzw. welche die Verwendung von nicht zugelassenen Elektroden im vorhinein verhindern. Dazu käme z.B. auch die RFID (radio frequency identification) gut in Frage. Auch jede andere Elektrode am Körper wäre (weniger sinnvoll) so auszuführen. Falls die Herstellung eines elektronischen Codes für die Elektroden zu aufwendig ist, könnte man das *FS*-EKG auch mit einem Barcode-Leser oder einem anderen Code-Leser ausstatten.

Darüber hinaus ist am Handgelenk eine Druckmanschette -32- gezeigt, die, zur konventionellen oszillometrischen Blutdruckmessung verwendet werden könnte, oder aber auch, wie in der Patentschrift (Europäische Patentanmeldung 05000042.1, Veröffentlichungsnummer 1522258 oder AT391.262B) ausgeführt, eine Flüssigkeitsgefüllte Blase über einer Arterie enthalten könnte. Diese kann mit einem variablen Druck beaufschlagt werden, so dass wie in obiger Patentschrift ausgeführt, eine automatische Blutdruckmessung und eine Pulswellenanalyse erfolgen kann. Daraus könnte dann unter anderem der so wichtige jetzt aber in der Routine nie gemessene zentrale Aortendruck errechnet werden. Dieser wichtige Parameter wird deswegen in der Praxis nie gemessen, da dies die zusätzliche Zeit von Personal beanspruchen würde. Die Mikroprozessor gesteuerte Blase (wie in der Patentschrift Europäische Patentanmeldung 05000042.1, Veröffentlichungsnummer 1522258 ausgeführt), erledigt dies jedoch im Hintergrund alleine, sodass dieser wichtige Parameter ohne zusätzlichen Personalaufwand gleichzeitig zu erzielen ist.

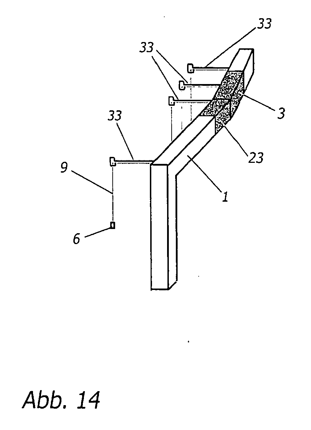

Wie in Abb. 14 gezeigt, könnte der Gerätearm -1- auch dazu benützt werden um von diesem aus die Kabel -9 - an den Patienten heranzuführen, wobei diese, um eine Verwirrung der Kabel -9- zu verhindern, wie dies sonst bei der Vielzahl der Kabel schwer vermeidlich ist, von unterschiedlichen Positionen am Gerätearm an den Körper herangeführt werden könnten (z.B. Beinelektroden von einem Ende des Gerätearms aus, Kopf und Brustkorbelektroden vom anderen Ende des Gerätearms aus). Im mit -1- gezeigten Gerätearm sind vorteilhaft auch die mechanischen oder elektronischen Umschalter-23-untergebracht, die vom zentralen Mikroprozessor, der vorteilhaft im zentralen *FS*-EKG -10-untergebracht ist, gesteuert, die jeweiligen Verbindungen vom *FS*-EKG -10- zu den Elektrodenpositionen -E1- bis -E14-, ev. bis E18 herstellen. Vom FS-EKG -10- zum Gerätearm -1- müssen im Minimalfall nur 2 Strom- bzw.. 2-Spannungskabel bzw. die Steuerleitungen bzw. Schirme führen, die das eventuell vorverstärkte Signal transportieren. Dies hat den Vorteil von noch besserer Übersichtlichkeit und vor allem, dass die im Gerätearm -1- untergebrachten berührungslosen Messgeräte -3- nicht durch die Kabel -9- gestört werden. Dazu könnte z.B. der Gerätearm auch mit Seitenstützen -33- für die Kabel-9- ausgeführt sein, welche die Kabel aus dem Messfeld der berührungslosen Messgeräte -3-fernhalten. Die vom Gerätearm -1- ausgehenden Kabel -9- hätten auch den Vorteil, dass die Kabel -9- zum Patienten , die vorteilhaft geschirmt sind, gleich lang sein können. Die Schirmung könnte auch als aktive Schirmung ausgeführt sein.

Sollte ein einzelnes Bündelkabel -9- verwendet werden, wie in Abbildung 8 und Abb. 9 gezeigt, von dem alle Elektrodenanschlüsse ausgehen, wird die Gefahr der Verwicklung des/der Kabel deutlich reduziert und die Seitenstützen könnten auch wegfallen. Um das Gerät auch transportierbar zu machen, wird vorgeschlagen alle.vom FS-EKG -10- und Messwagen -15- weg stehenden Teile, wie z.B., Gerätearm -1-, bzw. auch die Seitenstützen -30- entweder als Teleskopteile auszuführen, oder am besten in kurzen Segmenten an den Messwagen -15- heranschwenken zu können.

. Zur Distanzmessung zwischen den Elektroden allein könnte auch das zumindest eine berührungslose Messgerät -3-, an den am Körper aufgebrachten Elektroden -5a bis 5d- bzw. deren Elektrodenhalterungen -6a bis 6d- , mit denen der Kontakt zu den Kabel -9-, erfolgt, angebracht sein, wobei dieses als Distanzmessgerät (z.B. nach einer der oben angeführten Methoden) ausgeführt sein müsste (keine Abbildung).

Wie aus Abbildung 8 bis 14 ersichtlich, erfordert es eine große Zahl von unterschiedlichen Messungen an den ohnehin routinemäßig vorhandenen Elektroden, die nur durch zahlreiche Schaltvorgänge und/oder durch mehrere Impedanzgeräte zu bewerkstelligen sind. Die endgültige Ausprägung wird von technischen und finanziellen Gesichtspunkten abhängen.

Zu beachten ist jedenfalls, dass trotz der zahlreichen Ableitungen für jede einzelne der übliche für die Zulassung erforderliche Schutz für den Patienten und Untersucher. notwendig ist, um auch bei Applikation eines elektrischen Impulses z.B. im Falle einer Kardioversion bzw. Defibrillation bzw. auch bei einer Fehlfunktion des FS-ECGs -10- den Patienten nicht zu gefährden. Der Aufwand sowohl in technischer Hinsicht als auch der gegebene Zeitaufwand ist trotz der zahlreichen Schaltvorgänge trivial, da für die meisten Ableitungen nur die Grundimpedanz z0, aber nicht das dz bzw. dz/dt mit dem Herzschlag registriert werden muss.

Nachdem die Umschaltungen automatisch, am besten Mikroprozessor-gesteuert erfolgen, merkt weder Arzt noch Patient irgendetwas von der Komplexität der Methode und innerhalb von Minutenfrist kann das Ergebnis vorliegen. Auch kann aus dem Vergleich der gemessenen Impedanzen leicht erfasst werden, ob die Elektroden vertauscht wurden und eine entsprechende Warnung kann ausgegeben werden. Auch könnte durch unterschiedliche Konfiguration von Mehrfachsteckern gewährleistet werden, dass jeweils nur das richtige Kabel -9- an die richtige Elektrode angeschlossen werden kann. Die entsprechende konventionelle Farbgebung von Steckern und zugehörigen elastischen Klemmen (RA = rot, LA = gelb, LL = grün, RL = schwarz, und neu: linker Oberschenkel = hellgrün, rechter Oberschenkel = grau) macht die Durchführung auch für ungeschultes Personal problemlos.

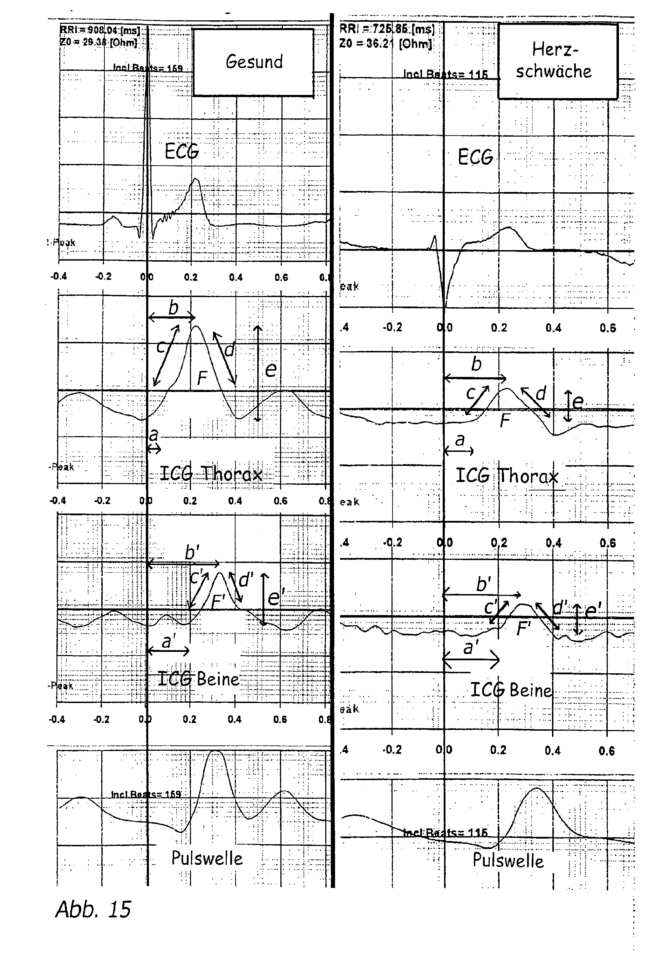

Abb. 15 zeigt das gleichzeitig am Thorax (oberer Teil der Abb.) und an den Beinen (unterer Teil der Abb.) registrierte Impedanzsignal. Auf der linken Seite der Abb. 15 sind die Signale bei einer gesunden Kontrolle (Healthy) aufgezeichnet. Wie ersichtlich kommt die Änderung der Impedanz (z) nach der Zeit mit dem Herzschlag, nämlich das dz/dt an den Beinen mit einem Intervall -a'- gegenüber dem Thorax -a- verspätet, wie man das auch physiologisch erwarten muss. Der Zeitunterschied des Steilanstieges von dz/dt zwischen dem Thorax und dem Beinsegment lässt wichtige Rückschlüsse auf die Herz- und Gefäßfunktion zu, da es bei Kongestion im peripheren Kreislauf zu einer Beeinflussung des Intervalls (a,a',b,b'), zu einer Beeinflussung der Kurvenform mit verändertem Anstieg -c- und -c'- und Abfalles -d- und -d'- des Impedanzsignals bzw. der Amplitude -e- und -e'- sowie der Fläche -F- und -F'- kommt. Die ist klar aus den Unterschieden zwischen dem gesunden Menschen (links) und dem Herzkranken auf der rechten Seite der Abbildung, die von einem Herzkranken stammt, auf den ersten Blick ersichtlich. Außerdem wird eine komplexe Signalanalyse mit Vermessung auch aller Flächen des Kurvenverlaufes hilfreich sein. Daher kann aus dem dz oder dz/dt Signal von Thorax *und* Beinen viel besser auf die tatsächliche Herzleistung geschlossen werden, als bei Messung des Thoraxsegmentes allein. Speziell kann der Teil des dz im Thorax, der durch die reflektierte Pulswelle verändert wird, erkannt und für diesen reflektierten Teil korrigiert werden. Die Ähnlichkeit von jeweiligem dz in Thorax und Beinen, lässt darauf schließen, dass die Interpretation des Thorax dz bisher viel zu kompliziert war. Dies lässt sich deswegen feststellen, da ohne Existenz von linkem und rechtem Herzen, Vorhöfen, großem und kleinem Kreislauf die Beinkurve so ähnlich der Thoraxkurve ist. Es könnte bei jetzt ja vorhandener genauer Kenntnis des Gesamtvolumens in einem Segment genügen das gesamte dz zu erfassen und nur den Teil des dz des Thorax abzuziehen, welcher aus den Beinen und anderen Teilen der Peripherie stammt, um genau das Schlagvolumen zu erhalten. Außerdem lässt sich erstmals auch ohne Verwendung von mechanischen Abnehmern rein elektrisch die Pulswellenlaufzeit in der Aorta genau vermessen, dazu hilft ebenfalls das berührungslose Messgerät -3- zur Messung der Körperoberfläche, weil die Distanz zwischen Thorax und Beinen millimetergenau vermessen werden kann. Nur die Pulswellenlaufzeit in der zentralen Aorta (jedoch nicht im Arm oder Beinsegment) ergibt wichtige Informationen über die Gefäßeigenschaft und korreliert mit der Prognose von Herz-Kreislauferkrankungen.

Aus den unterschiedlichen Gipfelhöhen des dz oder dz/dt bzw. auch aus einer Formalanalyse der Impedanzkurve des linken und rechten Beines können auch wichtige Rückschlüsse auf arterielle Durchblutung, Durchblutungsunterschiede der beiden Beine und auch Rückschlüsse über venöse Abflusshindernisse gezogen werden. Gegebenenfalls ließe sich dies auch mit Staumanschetten an den Beinen weiter verbessern, indem die Impedanzmessung als Venenverschlußplethysmographie ausgeführt ist. Eine Erhöhung des Druckes in der Manschette über den venösen Druck oder auch in die Gegend des arteriellen Druckes macht die Methode als plethysmographische oder oszillatorische Methode mit Hilfe der genauen Volumsmessung noch vielseitiger.

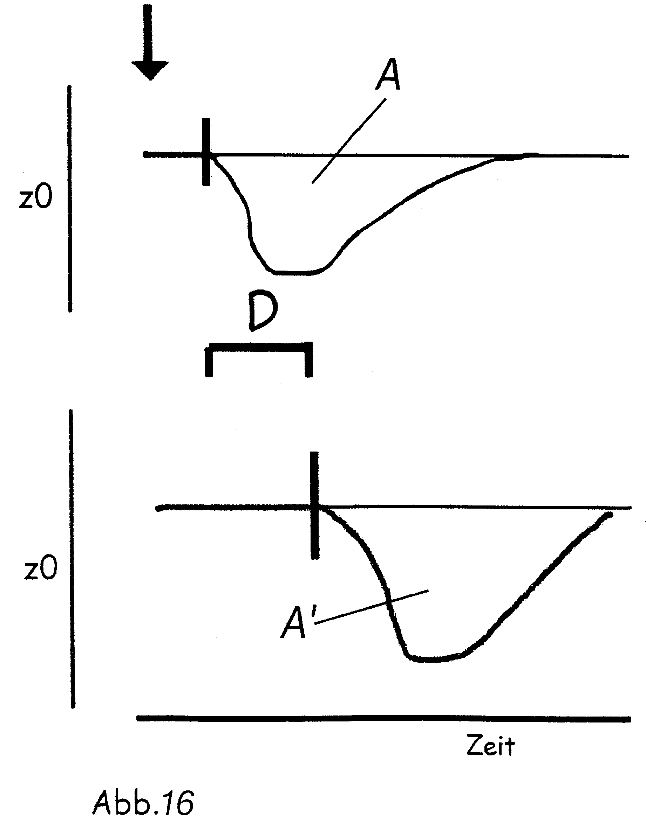

In der Abb. 16 wird die Änderung der Impedanz im oberen Thoraxsegment und im unteren Thoraxsegment nach intravenöser Injektion eines Bolus einer Flüssigkeit in eine Armvene, welche die Impedanz des Blutes ändert, gezeigt. Zur Messung dieses Impedanzsprunges könnten z.B. auch zur Einspeisung des Stroms die Nackenelektrodenposition -E1- und die Beinelektrodenposition -E12 und E14 - (siehe Abb. 10 und 12) herangezogen werden, die Brustwandelektroden V1 bis V6 könnten wahlweise zur Messung des Impedanzsprunges herangezogen werden. Es findet sich z.B. ein Zeitversetzter Impedanzsprung zwischen V1 und V2 einerseits und zwischen V5 und V6 andererseits, der mit -D- gekennzeichnet ist und der ein Maß für die Herzleistung darstellt. Auch die Fläche unter der jeweiligen Kurve -A- und -A'- , bzw. die Kurvenform, ausgelöst durch die Injektion des Mediums mit unterschiedlicher Leitfähigkeit im Verhältnis zu Blut gibt wesentliche Auskünfte über die Herzfunktion entsprechend dem Fick'schen Prinzip. Auch andere Segmente des Körpers könnten zur Messung des Impedanzsprunges und der Kurvenform herangezogen werden Eine Verwendung dieses Prinzips in anderen zusätzlich frei wählbaren Regionen, z.B. auch im Beinsegment bzw. auch im Bereich der arteriellen Strombahn ist möglich.

Es ist klar, dass bei der äußersten Komplexizität der menschlichen Kompartmente einerseits zur Definierung derselben die vorgeschlagenen multiplen Messungen notwendig sind und dass andererseits exzellente klinische Evaluations- und Eichungsdaten an einer großen Zahl von Patienten notwendig sind, um die Methode in die Klinik einführen zu können. Die Verbesserung der Abschätzung von komplexen Körperräumen wird sehr anschaulich in Abb.2 bis Abb. 7 vor Augen geführt. Nur das beste Ergebnis mit Einsatz der multiplen Messpunkte bei mehreren Frequenzen und die gleichzeitige Kenntnis der wahren Dimension des untersuchten Körperabschnittes ist, wie in Abb. 7 dargestellt, hinreichend genau um klinisch brauchbar zu sein.

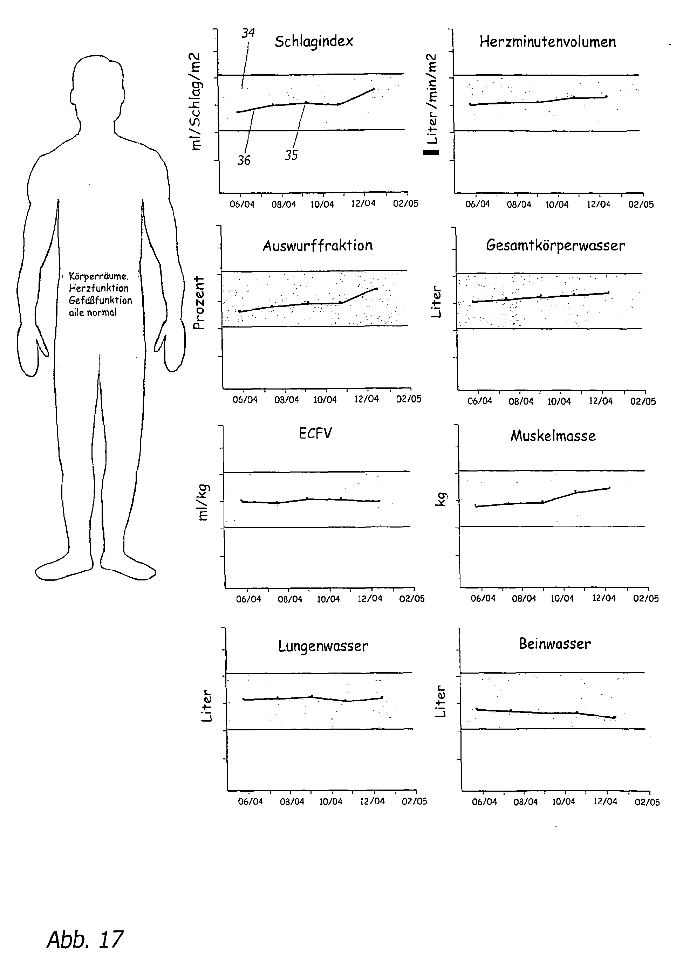

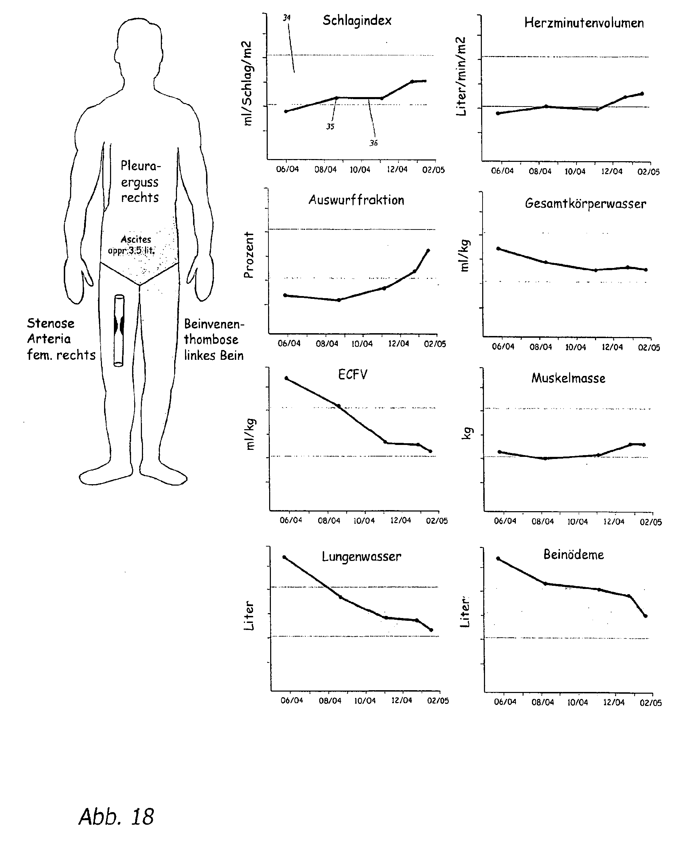

Abb. 17 und Abb.18 zeigen einen Beispielweisen Ausdruck eines Function&Spaces (*FS*)-EKGs, aus dem ersichtlich ist, dass die wichtigen haemodynamischen und Flüssigkeitsdaten in numerischer und graphischer Form und auch als Textbaustein untergebracht werden können. So kann bei gleichzeitiger Speicherung des Patienten in einer Datenbank sofort ein Vergleich mit den Vormessungen angestellt werden, der sich besonders auch in graphischer Form bewährt. Dabei können z.B. auch die Normwerte in Form von Normfeldern -34- angezeigt sein, in welche die Patientenwerte als Messpunkte-35- bzw. bei Verlaufsbeobachtungen als durch Verbindungslinien -36-miteinander verbundene Messpunkte -35- angezeigt werden können, wobei z.B. auf der x-Achse die Zeitachse und auf der y-Achse die Messwertgröße aufscheint. So kann auf einen Blick der Erfolg der durch den Arzt eingeleiteten Therapie abgeschätzt bzw. auch in der Folge die Therapie weiter verbessert werden.

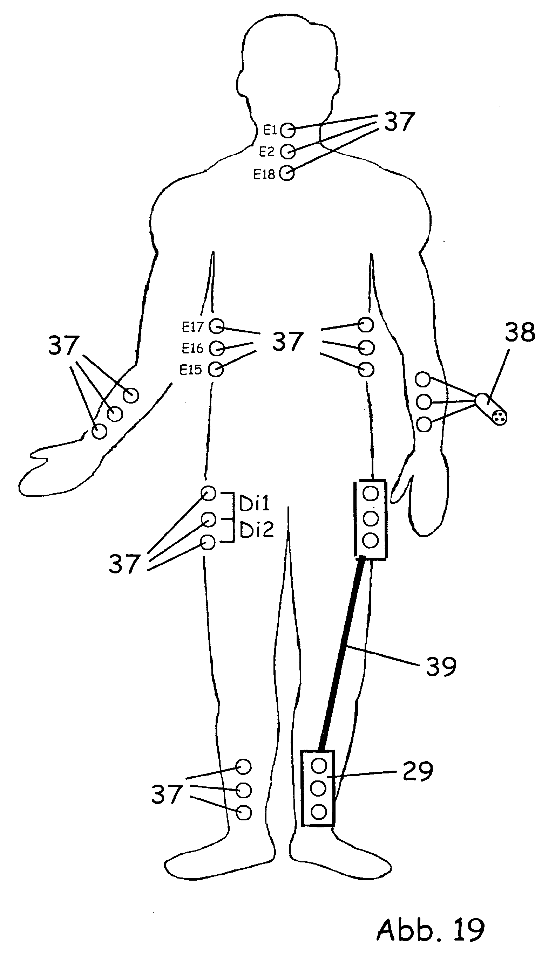

Abb. 19 zeigt eine weitere bevorzugte Elektrodenausführung bzw. Positionierung am menschlichen Körper. Dabei sind alle Begrenzungen des jeweils untersuchten Segmentes z.B. mit Dreifachelektrodenelementen -37- besetzt. Diese Elektrodenanordnung macht es möglich den Strom jeweils nur in das gerade untersuchte Segment einzubringen und gleichzeitig die Länge des zwischen den Spannungselektroden gemessenen Segmentes an beiden Enden gering zu variieren. Mit dieser Anordnung bekommt man die sogenannten "Randzonenphänomene" noch besser in den Griff. Ideal wäre es nämlich ein Segment so zu untersuchen, dass man in die äußeren gedachten Schnittflächen des untersuchten Segmentes gleichmäßig, über die gesamte Fläche verteilt, Elektroden einbringt, die für die Spannungsmessung herangezogen werden. Dies ist beim menschlichen Körper natürlich nicht möglich, ohne ihn zu verletzen und so man muss sich mit Oberflächenelektroden auf der Haut begnügen. Am Rand des Segmentes gelingt es jedoch so nur einen Teil der im Segment wirksamen Spannung abzugreifen, naturgemäß besonders wenig, wenn unter den Spannungselektroden schlecht leitende Gewebeanteile, wie Sehnen, Knochen oder Fettgewebe liegen. Wenn der Strom hingegen an der selben Stelle eingebracht wird, an dem die Spannung abgegriffen wird, wird ein größerer und repräsentativerer Teil der Spannung abgegriffen. So kann mit 9 bis 11 Dreifachelektroden ein äußerst repräsentatives Bild der Flüssigkeitsverteilung im Körper gewonnen werden. Natürlich könnte auch das Bein durch weitere Elektrodenelemente in ein Oberschenkel und Unterschenkelsegment unterteilt werden, bzw. nur Unterschenkel oder Oberschenkel gemessen werden. Die zahlreichen Umschaltvorgänge (mit bis zu 10 oder mehr verschiedenen Stromeinspeisungen und bis zu 30 oder mehr unterschiedlichen Spannungsmessungen) erfolgen unbemerkt vom Nutzer vollautomatisch. Auch eine Ausführung der in Abb. 19 gezeigten Elektroden als Bandelektroden ist günstig. Auch eine Ganzkörperimpedanzmessung mit dem Vorteil der berührungslosen Volumetrie ist natürlich möglich Die Abstände zwischen den Elektroden die ein Segment begrenzen, werden ebenfalls unbemerkt und vollautomatisch erfasst. Es ist offensichtlich von Vorteil, jede der dargestellten Elektrodenpositionen bestehend aus einer Dreifachelektrode -37- jeweils nur mit einer unverwechselbaren Steck- oder Klemmverbindung (z.b. Mehrfachstecker -38-) auszustatten. Der Abstand der Elektroden an jeweilig einer Begrenzung des Segmentes ergibt sich entweder a) durch die Anbringung der Elektroden auf dem gemeinsamen Träger -29-. Dabei wird z.b. auch auf die Ausführung als- Saugelektrode -30- in Abb. 13 oder elastische Klemme -25- in Abb. 11- verwiesen, wobei lediglich statt den Zweifachelektroden nun Dreifachelektroden vorliegen. Andererseits kann ebenfalls automatisch durch das verwendete Verfahren der Oberflächendarstellung, z.B. durch die 3D Photogrammometrie automatisch die Distanzen Di1 und Di2 der Elektroden innerhalb des Dreifachelektrodenelementes -37- ermittelt werden, dann wäre auch kein gemeinsamer Träger -29- für die Elektroden notwendig. Auch ein Abstandhalter -39- für die jeweils zwei Doppelektroden -22- oder Dreifachelektrodenelemente -37,- die das gerade untersuchte Segment begrenzen, wäre vorstellbar, dann wäre auch dadurch der Oberflächenabstand der Spannungselektroden vom oberen zum unteren Ende des Segmentes bekannt, ohne dass dieser durch das bildgebende Verfahren ermittelt werden muss. Beispielsweise wird die Untersuchung für alle dargestellten oder auch zusätzlichen Segmente (zB. Oberschenkel und Unterschenkel separat) an Hand der Elektrodenpostionen E1, E2, E15, E16, E17 und E18 dargestellt. Der Strom wird hier beispielsweise in der Position E1 und E15 eingeleitet und die Spannung wahlweise zwischen E16 und E2, oder zwischen E17 und E2, oder zwischen E16 und E18, oder zwischen E17 und E18 gemessen. Damit lässt sich dann auch z.B. eine "elektrisch operative Länge" wie in der Arbeit von Skrabal et al ausgeführt, ermitteln und das elektrisch leitende Volumen in einem Segment noch genauer errechnen⁷. Alle anderen Segmente in Abb. 19 oder auch zusätzliche Segmente (z.B. Oberschenkel und Unterschenkel getrennt) können analog zu dem einen dargestellten Segment vermessen werden. Bei der Berechnung der Volumina der in Abb. 19 dargestellten Segmente kommen alle Vorteile einer rein physikalischen Gesetzen unterliegenden Berechnung ohne jede Annahme voll zum tragen: So wird die Segmentlänge automatisch und fehlerfrei erkannt; es wird der Querschnitt des Segmentes millimetergenau und scheibchenweise gemessen, was für den Widerstand des Segmentes von entscheidender Bedeutung ist, weil z.B. eine Verjüngung des Segmentes z.B. im Kniegelenk eine unverhältnismäßig große Erhöhung des Widerstandes bewirkt, was nach exakten physikalischen Formeln genau berechnet werden kann; es kann durch den Frequenzsweep z.B. durch den "Cole-Cole Plot" der Widerstand bei der Frequenz Null und bei der Frequenz Unendlich genau berechnet werden¹⁴, und es kann durch die "Hanai Mixture Theorie¹⁵", z.B. auch die "Second Generation Hanai Mixture Theorie¹⁶" exakt physikalisch der Einfluss von geformten Elementen (den Körperzellen) auf den Widerstand des Segmentes berechnet werden. Auch der Widerstand eines Segmentes entsprechend der Formel
R_{S}= ρ*(L₁/A₁+L₂/A₂+L₃/A₃ bis Lₙ/Aₙ),
wobei R_{S} = Widerstand des Segmentes,
p = spezifische Widerstand des Leiters,
L₁ bis Lₙ = Längen der jeweils untersuchten Scheibchen des Segmentes
A₁ bis Aₙ = Fläche der jeweils untersuchten Scheibchen des Segmentes ,
kann nun erstmals unter Verwerfung der falschen Annahme von zylindrischen Leitern (noch dazu mit geschätzter kreisförmiger Durchschnittsfläche) mathematisch korrekt interpretiert werden. Bei Mitverwendung der nun auch bekannten Anatomie des Segmentes (siehe unten) könnte auch jedem Scheibchen sein der Anatomie angepasster spezifischer Widerstand (p) zugewiesen werden. Damit handelt es sich nicht mehr um eine empirische Schätzung, sondern um eine nachvollziehbare Methode, die rein physikalisch begründet ist. Durch die genaue volumetrische Vermessung des Körpers würde auch die Ganzkörperimpedanzmessung wesentlich verbessert, weil der bei dieser Methode bisher verwendete grobe Korrekturfaktor¹⁷ für die unterschiedlichen Durchmesser von Armsegment, Rumpf- und Beinsegment durch eine genau gemessene physikalische Größe ersetzt werden könnte, weil die scheibchenweise gemessenen Durchmesser von Arm, Rumpf und Bein erstmals genau bekannt sind. Auch kann jetzt erstmals die Kenntnis der Anatomie in das theoretische Modell eingehen: Als kleines Beispiel sei genannt, dass das Knie als solches durch die Volumetrie identifiziert werden kann. Nun ist bekannt, dass auf Höhe des Kniegelenkes fast ausschließlich, Knochen, Knorpel und Bindegewebe vorhanden sind, alles Gewebe mit sehr schlechter Leitfähigkeit, andererseits proximal und distal des Kniegelenkes große Muskelmassen mit sehr guter Leitfähigkeit vor allem bei hohen Frequenzen. In Hinkunft wird also durch die Kenntnis der Anatomie dieselbe in die Berechnung von Intra- und Extrazellulärwasser auf Grund des praktischen anatomischen Modells eingehen können. Bei der Zahl der verwendeten Elektroden bewährt es sich besonders, dass nur vom Hersteller des *FS*-EKG ausgelieferte Elektroden verwendet werden, da diese ja als Wegwerfartikel einen ständigen Umsatz garantieren. Daher könnte es sich bewähren, die Elektroden selbst, bzw. deren Verpackung mit einem Code zu versehen, der z.B. auch händisch in das FS-EKG Gerät eingegeben werden muss. Nur bei Übereinstimmung der Code-Nummer mit den im FS-EKG eingespeicherten Codenummer wäre dann das Gerät betriebsbereit.
¹⁴ Cole KS, Cole RH Dispersion and absorption in dielectrics I. Alternating current characteristics J Chem Phys 9: 341- 51, 1941
¹⁵ Hanai T. Electrical properties of emulsions. In: Emulsion Science, ed Sherman PH . London Academic, p 354- 477, 1968
¹⁶ Mathie JR. Second generation mixture theory equation for estimating intracellular water using bioimpedance spectroscopy. J Appl Physiol. 99: 780-81, 2005
¹⁷ De Lorenzo A, Andreoli A, Matthie J, Withers P. Predicting body cell mass with bioimpedance by using theoretical models: a technological review. J Appl Physiol 82: 1542-58, 1997

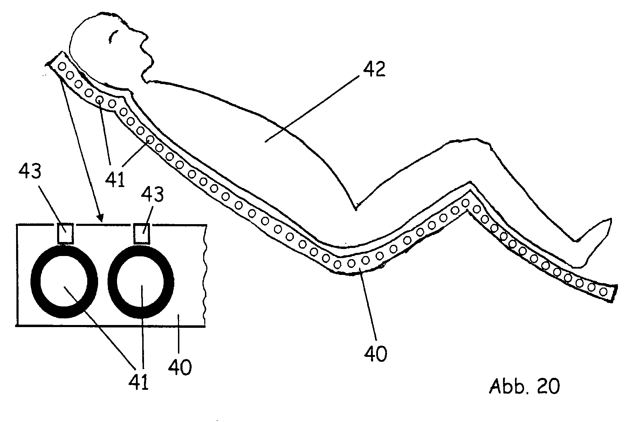

Weiters kann eine spezielle Ausführungsform der Oberfläche, auf welcher der Patient liegt. von Vorteil sein: Diese Ausführungsform kann entweder Bestandteil Untersuchungsliege -2- sein oder sie ist auch dann besonders günstig, wenn der Patient nicht aus seinem Bett auf eine Untersuchungsliege gehoben werden kann oder soll. Abb. 20 zeigt eine verformbare Matte -40-, deren Verformung definiert und ermittelbar ist, unter den Patienten gebracht, z.B. gerollt oder geschoben, der damit in dem Bett in dem er liegt, verbleiben kann.. Besonders günstig ist, wenn die Matte in der Querrichtung zum menschlichen Körper nicht oder nur gering verformbar ist und andererseits in der Längsrichtung des Körpers gut verformbar ist. Damit kann die Unterlage der Beugung und Streckung in den verschiedenen Gelenken (Kniegelenk, Hüfte, Wirbelsäule) gut folgen. Der seitlich neben dem untersuchten Lebewesen -42-herausragende Teil der Matte -40- wird durch das berührungslose bildgebende Verfahren genau erkannt und als Referenzfläche verwendet. (siehe Abb. 9). Wenn diese Referenzfläche repräsentativ auch für den unter dem menschlichen Körper liegenden Teil der Referenzfläche ist, kann die hintere, nicht dargestellt Begrenzung des menschlichen Körpers genau ermittelt werden. Abb. 20 zeigt eine mögliche Ausbildungsform der Matte im Querschnitt: Dabei werden bevorzugt runde, dreieckige, oder mehreckige Stäbe -41-, die sehr stabil sind (z.B. Rohre -41-) in eine verformbare Matte -40- z.B. aus geschäumtem Kunststoff eingebracht. Damit kann die Matte -40- den Krümmungen des Körpers folgen, und trotzdem ist durch die seitlich neben dem untersuchten Lebewesen herausragende Fläche der Matte die hintere Begrenzung des menschlichen Körpers genau bekannt, weil diese Matte -40- mit Hilfe der berührungslosen Messgeräte genau als Referenzfläche identifiziert ist (siehe Abb. 9) . Z.B. sind mit Hilfe von 4 Stereo Kameras (bestehend z.B. 8 CCDs) (siehe Abb. 9) sowohl die als Referenzfläche dienende verformbare Matte -40- als auch das untersuchte menschliche Lebewesen genau dreidimensional erfassbar. Unter der Matte befindet sich die konventionelle Liege oder das Bett, auf welcher der Patient liegt, die Form der Matte ist durch das Gewicht des Untersuchten bestimmt, wenn unterhalb der Liege, weiches verformbares Materialvorhanden ist (z.B. Schaumstoffe, weiche Polster u.s.w., nicht dargestellt). Eine andere Ausführungsform könnte darin bestehen, dass die Verformung in der Querachse der verformbaren Matte -40- genau definiert möglich ist. Sollten z.B. die Stäbe einen definierten Biegungsradius haben, könnte man auch aus den seitlichen neben dem untersuchten Körper herausragenden Stäben oder Rohren -40-, die Krümmung unterhalb des untersuchten Körpers berechnen. Dies hätte den Vorteil, dass sich die verformbare Matte -40- noch besser den Körperkonturen anschmiegt und damit die Begrenzung des untersuchten Körpers in dem Bereich, der durch die berührungslosen Messverfahren nicht dargestellt werden kann, durch die Krümmung der verformbaren Matte klar definiert ist. Die Anordnung der berührungslosen Messgeräte eine schließt ja Rundumdarstellung des untersuchten Körpers aus. So kann aber im sichtbaren Bereich die berührungslosen Messgeräte -2- und im auf der verformbaren Matte -40- die definierte Krümmung das Volumen des untersuchten Körpers und seiner Segmente definieren. Neben den verformbaren Stäben -41- sind natürlich andere Hilfsmittel für eine definierte Verformung denkbar. Nach Gebrauch kann die Matte zusammengerollt und auch leicht gereinigt werden. Damit ist eine Untersuchung des Menschen überall wo er gerade liegt, möglich. Auch die Ausstattung der Oberfläche der verformbaren Matte -40- mit zahlreichen innerhalb der Matte gleichmäßig verteilten Drucksensoren -43-, aus denen das Gesamtgewicht des Patienten ermittelt wird ist von Vorteil, weil aus dem Volumen und dem Gewicht die Dichte des menschlichen Körpers und damit auch sofort sein Fett- und Nichtfettanteil ermittelt werden kann. Eine andere Ausführungsform der Matte -40- könnte eine sogenannte "intelligente" oder "smarte" stark verformbare Matte sein, die ihre eigene Verformung gut erkennt und die sich lückenlos der hinteren nicht abbildbaren Kontur des Körpers anpasst, Diese Matte könnte z.B. aus leitfähigem Schaumstoff (z.B. bevorzugte Leitfähigkeit in der Tiefe der Matte) gefertigt sein, wobei durch die Verformung die elektrischen Eigenschaften der Matte verändert werden. Z.B könnte der durch die Kompression veränderte Widerstand in der Sagitallebene (in der Tiefenebene der Matte) bzw. auch veränderte Kapazitäten registriert werden, oder aber auch die Dehnung der Oberfläche die durch die Kompression der Matte an spezifischen Punkten entsteht. So ist z.B. sogenannter "conducting foam" am Markt, der z.B. aus Polyether-Polyurethan hergestellt und mit strukturiertem Kohlenstoff imprägniert ist und dessen Widerstand sich ändert, wenn er komprimiert oder gedehnt wird. Der Kohlenstoff wird z.B. durch ein synthetisches polymeres Latex an die z.B. offene Schaumstruktur gebunden. Es könnte auch ein in sich leitfähiges Material wie Polypyrrol (Ppy) zum Beschichten des z.B. offenen Schaumstoffes verwendet werden. Eine Zusammensetzung der Matte aus vielen kleinen Einzelelementen von leitfähigem Material z.B. voneinander isoliert, ist denkbar.

Man wird natürlich ein Material mit sehr guter und reversibler Verformbarkeit wählen. Etwaige Änderungen der elektrischen Eigenschaften der "smarten" Matte mit geänderter Temperatur müssten natürlich beachtet werden. Es wird günstig sein, die Matte gegenüber dem Körper und gegenüber der Unterlage mit einer dünnen sehr gut elektrisch isolierenden Schicht auszustatten.. Es ist auch daran gedacht jedes auch in Zukunft in Entwicklung befindliche oder kommerziell erhältliche Verfahren dafür anzuwenden. So entsteht ein lückenloses 3D-Bild von der Oberfläche des Körpers im nicht sichtbaren Teil des Körpers, der aufliegt, geliefert von der "intelligenten" oder "smarten" Matte als sogenanntes "Negativphantom", im sichtbaren Teil des Körpers geliefert von der berührungslosen 2D oder 3D -Darstellung der Körperoberfläche.. Wenn die Matte-40- auch einen definierten elastischen Modulus aufweist, ergibt sich aus der Verformung auch das Körpergewicht des Patienten ohne separate Drucksensoren.

## Patentansprüche

1. Gerät zur elektrischen Messung von Körperfunktionen und Zuständen, wie insbesondere z.B. der elektrischen und mechanischen Herztätigkeit, der Durchblutung von Körperteilen, des Volumens, der Volumenänderung und der Zusammensetzung von elektrisch leitenden Körperflüssigkeiten, wobei das Gerät adaptiert ist, mittels am Körper befestigbarer Elektroden elektrische Messgrößen zu erfassen, die mit den Körperfunktionen und Zuständen korrespondieren und aus den erfassten elektrischen Messgrößen die Körperfunktionen und Zustände zu errechnen, **dadurch gekennzeichnet, dass** das Gerät adaptiert ist, besagte Körperfunktionen und Zustände mittels Messung der Impedanz und deren Änderung mit der Zeit von verschiedenen annähernd zylindrischen oder ovalen Körpersegmenten mit relativ konstantem Querschnitt entweder sequentiell oder gleichzeitig an verschiedenen nachgeschalteten Körpersegmenten, in zumindest einer Richtung zu ermitteln.

2. Gerät nach Anspruch 1, **dadurch gekennzeichnet, dass** in zumindest einem der Segmente die Grundimpedanz bei zwei Frequenzen gemessen wird, dass daraus ein Parameter für das Verhältnis bzw. die Verteilung von intra- zu extrazellulärem Wasser errechnet wird und dass aus den kombinierten Messgrössen ein Parameter für die Herzleistung errechnet wird.

3. Gerät nach einem der vorhergehenden Ansprüche, **dadurch gekennzeichnet, dass** zwei für die Messung der Herzleistung verwendete Körpersegmente durch ein dazwischen liegendes Körpersegment voneinander getrennt sind.

4. Gerät nach einem der vorhergehenden Ansprüche, **dadurch gekennzeichnet, dass** es sich bei dem Parameter für die Herzleistung um die NYHA Klasse handelt.

5. Gerät nach einem der vorhergehenden Ansprüche, **dadurch gekennzeichnet, dass** es sich bei dem Parameter für die Herzleistung um einen biochemischen (z.B. hormonellen) Parameter, wie z.B. BNP handelt und/oder dass die Steifigkeit des Herzmuskels und somit die diastolische Funktion als Parameter ausgegeben wird.

6. Gerät nach einem der vorhergehenden Ansprüche, **dadurch gekennzeichnet, dass** es über die Elektroden einen Wechsel-Messstrom von zumindest einer Frequenz in den Körper einbringen kann und in vom Wechsel-Messstrom durchflossenen Körpersegmenten die Grundimpedanz (z0) und/oder ihre Komponenten Wirkwiderstand, Blindwiderstand und/oder Phasenwinkel bzw. deren Änderung oder Ableitung über die Zeit erfasst.

7. Gerät nach einem der vorhergehenden Ansprüche, **dadurch gekennzeichnet, dass** die Impedanzänderung mit dem Herzschlag und die Grundimpedanz (z0) in drei seriell geschalteten Körpersegmenten gemessen wird, von denen zumindest ein Körpersegment aus zwei parallel-geschalteten Teilsegmenten, wie z.B. Arme oder Beine, zusammengesetzt ist, wobei vorzugsweise die Elektroden an einem jeweiligen Teilsegment abschaltbar sind.

8. Gerät nach einem der vorhergehenden Ansprüche, **dadurch gekennzeichnet, dass** es die Grundimpedanz (z0) und die Impedanzänderung mit dem Herzschlag, und/oder deren Ableitung bei mehr als einer Frequenz misst.

9. Gerät nach einem der vorhergehenden Ansprüche, **dadurch gekennzeichnet, dass** das linke und das rechte Bein separat vermessen werden und daraus Parameter über arterielle und venöse Durchblutungsstörungen und/oder ein Parameter für das Verhältnis von extrazu intrazellulärem Wasser ermittelt werden.

10. Gerät nach einem der Ansprüche 2 bis 9, **dadurch gekennzeichnet, dass** ein Parameter für Wasseransammlung bzw. das Verhältnis von extra- zu intrazellulärem Wasser im dazwischen geschalteten Körpersegment, z.B. Ascites, ermittelt wird.

11. Gerät nach einem der vorhergehenden Ansprüche, **dadurch gekennzeichnet, dass** es zumindest eine Halselektrode oder eine Kopfelektrode, zumindest drei Thoraxelektroden, zumindest zwei distale Extremitätenelektroden und zumindest eine proximale Extremitätenelektrode aufweist, wobei vorzugsweise die Halselektrode, Kopfelektrode und Thoraxelektroden als Bandelektroden, Doppelbandelektroden, Saugelektroden oder punktförmige Klebeelektroden ausgebildet sind, und wobei vorzugsweise die Extremitätenelektroden als Bandelektroden, Doppelbandelektroden oder Doppelklemmen ausgebildet sind.

12. Gerät nach Anspruch 11, **dadurch gekennzeichnet, dass** Elektroden als Doppelektroden (22) oder Dreifachelektrodenelemente (37), die jeweils auf einem gemeinsamen Träger (29) bzw. gemeinsamen Bügel (26) in fixem Abstand aufgebracht sind, ausgebildet sind.

13. Gerät nach einem der vorhergehenden Ansprüche, **dadurch gekennzeichnet, dass** es ein EKG Gerät umfasst.

14. Gerät nach Anspruch 13, **dadurch gekennzeichnet, dass** Impedanzelektroden (E1-18) zumindest teilweise auch als EKG Elektroden (RA, LA, RL, LL, V1-V6, V5r, V6r) beschaltbar sind.

15. Gerät nach einem der vorhergehenden Ansprüche, **dadurch gekennzeichnet, dass** elektrische Relais oder elektronische Umschalter (23) für die Umschaltung des Stromes zu und von den Elektroden und für die Impedanzmessung vorgesehen sind.

16. Gerät nach einem der vorhergehenden Ansprüche, **dadurch gekennzeichnet, dass** die Frequenz des Wechselstroms von einer unteren Messfrequenz bis zu einer oberen Messfrequenz kontinuierlich verändert wird.

## Claims

1. A device for the electrical measurement of bodily functions and conditions such as in particular the electrical and mechanical action of the heart, the circulation through body parts, the volume, the volume change and the composition of electrically conductive body fluids, wherein the device is adapted for detecting electrical measurands corresponding to the bodily functions and conditions via electrodes attachable to the body and for calculating the bodily functions and conditions from the detected electrical measurands, **characterized in that** the device is adapted for determining said bodily functions and conditions by measuring the impedance of different roughly cylindrical or oval body segments having relatively constant cross-sections and the change over time thereof either sequentially or simultaneously on different series-connected body segments in at least one direction.

2. A device according to claim 1, **characterized in that** the basic impedance is measured at two frequencies in at least one of the segments, that a parameter for the ratio or the distribution, respectively, of intracellular to extracellular water is calculated therefrom and that a parameter for cardiac performance is calculated from the combined measurands.

3. A device according to any of the preceding claims, **characterized in that** two body segments used for the measurement of the cardiac performance are separated by an intermediate body segment.

4. A device according to any of the preceding claims, **characterized in that** the parameter for the cardiac performance belongs to the NYHA class.

5. A device according to any of the preceding claims, **characterized in that** the parameter for the cardiac performance is a biochemical (e.g., hormonal) parameter such as, e.g., BNP and/or that the stiffness of the heart muscle and thus the diastolic function are output as a parameter.

6. A device according to any of the preceding claims, **characterized in that,** via the electrodes, it can introduce an alternating measurement current of at least one frequency into the body and detects the basic impedance (z0) and/or its components active resistance, reactance and/or phase angle and the alteration or derivative thereof, respectively, over time in body segments passed through by the alternating measurement current.

7. A device according to any of the preceding claims, **characterized in that** the change in impedance with the heartbeat and the basic impedance (z0) are measured in three series-connected body segments, of which at least one body segment is composed of two partial segments connected in parallel, such as, e.g., arms or legs, wherein the electrodes can preferably be switched off on a respective partial segment.

8. A device according to any of the preceding claims, **characterized in that** it measures the basic impedance (z0) and the change in impedance with the heartbeat and/or the derivative thereof at more than one frequency.

9. A device according to any of the preceding claims, **characterized in that** the left leg and the right leg are measured separately and parameters regarding arterial and venous perfusion disturbances and/or a parameter for the ratio of extracellular to intracellular water are determined therefrom.

10. A device according to any of claims 2 to 9, **characterized in that** a parameter for water retention and the ratio of extracellular to intracellular water, respectively, in the interposed body segment, e.g., ascites, is determined.

11. A device according to any of the preceding claims, **characterized in that** it comprises at least one neck electrode or a head electrode, at least three thorax electrodes, at least two distal extremities electrodes and at least one proximal extremities electrode, wherein the neck electrode, the head electrode and the thorax electrodes are preferably designed as band electrodes, double band electrodes, suction electrodes or punctiform adhesive electrodes and wherein the extremities electrodes are preferably designed as band electrodes, double band electrodes or double clamps.

12. A device according to claim 11, **characterized in that** electrodes are designed as double electrodes (22) or triple electrode elements (37), which, in each case, are applied to a common support (29) or to a common bracket (26), respectively, at a fixed distance.

13. A device according to any of the preceding claims, **characterized in that** it comprises an ECG device.

14. A device according to claim 13, **characterized in that** impedance electrodes (E1-18) can be wired at least partially also as ECG electrodes (RA, LA, RL, LL, V1-V6, V5r, V6r).

15. A device according to any of the preceding claims, **characterized in that** electrical relays or electronic change-over switches (23) are provided for switching the current to and from the electrodes and for the impedance measurement.

16. A device according to any of the preceding claims, **characterized in that** the frequency of the alternating current is continuously altered from a lower measuring frequency to an upper measuring frequency.

## Revendications

1. Appareil pour la mesure électrique des fonctions du corps et d'états, comme en particulier par exemple l'activité électrique et mécanique du coeur, l'irrigation sanguine des parties du corps, le volume, la variation du volume et la composition des fluides corporels électro-conducteurs, l'appareil étant adapté à détecter des grandeurs de mesure électriques au moyen d'électrodes pouvant être fixées sur le corps, qui correspondent aux fonctions du corps et aux états, et à calculer les fonctions du corps et les états à partir des grandeurs de mesure électriques détectées, **caractérisé en ce que** l'appareil est adapté à déterminer lesdites fonctions du corps et les états par mesure de l'impédance et de sa variation avec le temps de différents segments du corps presque cylindriques ou ovales et présentant une section transversale relativement constante soit de manière séquentielle soit de manière simultanée sur différents segments corporels situés en aval, dans au moins une direction.

2. Appareil selon la revendication 1, **caractérisé en ce que** dans au moins un des segments, l'impédance de base est mesurée à deux fréquences, **en ce qu'**un paramètre pour le rapport ou la répartition de l'eau intracellulaire à l'eau extracellulaire est calculé et **en ce qu'**un paramètre pour la puissance cardiaque est calculé à partir des grandeurs de mesure combinées.

3. Appareil selon l'une quelconque des revendications précédentes, **caractérisé en ce que** deux segments corporels utilisés pour la mesure de la puissance cardiaque sont séparés l'un de l'autre par un segment corporel situé entre ces derniers.

4. Appareil selon l'une quelconque des revendications précédentes, **caractérisé en ce que** le paramètre pour la puissance cardiaque est la classification NYHA.

5. Appareil selon l'une quelconque des revendications précédentes, **caractérisé en ce que** le paramètre pour la puissance cardiaque est un paramètre biochimique (par exemple hormonal), tel que par exemple BNP et/ou **en ce que** la rigidité du muscle cardiaque et donc la fonction diastolique est délivrée en tant que paramètre.

6. Appareil selon l'une quelconque des revendications précédentes, **caractérisé en ce qu'**il peut introduire un courant de mesure alternatif d'au moins une fréquence dans le corps par l'intermédiaire des électrodes et l'impédance de base (z0) et/ou ses composantes résistance effective, réactance et/ou angle de phase ou leur variation ou leur dérivée au fil du temps sont détectées dans les segments corporels traversés par le courant de mesure alternatif.

7. Appareil selon l'une quelconque des revendications précédentes, **caractérisé en ce que** la variation d'impédance est mesurée avec le rythme cardiaque et l'impédance de base (z0) est mesurée dans trois segments corporels situés en série, parmi lesquels au moins un segment corporel est composé de deux segments partiels situés en parallèle, par exemple les bras ou les jambes, les électrodes pouvant de préférence être désactivées sur un segment partiel respectif.

8. Appareil selon l'une quelconque des revendications précédentes, **caractérisé en ce qu'**il mesure l'impédance de base (z0) et la variation d'impédance avec le rythme cardiaque, et/ou sa dérivée en présence de plus d'une fréquence.

9. Appareil selon l'une quelconque des revendications précédentes, **caractérisé en ce que** la jambe gauche et la jambe droite sont mesurées séparément et des paramètres concernant le manque d'irrigation sanguine artérielle et veineuse et/ou un paramètre pour le rapport de l'eau extracellulaire à l'eau intracellulaire en sont déduits.

10. Appareil selon l'une quelconque des revendications 2 à 9, **caractérisé en ce qu'**un paramètre pour la rétention d'eau ou le rapport de l'eau extracellulaire à l'eau intracellulaire dans le segment corporel situé entre celles-ci, par exemple des ascites, est déterminé.

11. Appareil selon l'une quelconque des revendications précédentes, **caractérisé en ce qu'**il comprend au moins une électrode de cou ou une électrode en cuivre, au moins trois électrodes de thorax, au moins deux électrodes d'extrémité distales et au moins une électrode d'extrémité proximale, l'électrode de cou, l'électrode en cuivre et les électrodes de thorax étant de préférence réalisées sous la forme d'électrodes à bande, d'électrodes à double bande, d'électrodes d'aspiration ou d'électrodes auto-adhésives en forme de point, et les électrodes d'extrémité étant de préférence réalisées sous la forme d'électrodes à bande, d'électrodes à double bande ou de doubles pinces.

12. Appareil selon la revendication 11, **caractérisé en ce que** les électrodes sont réalisées sous la forme d'électrodes doubles (22) ou d'éléments d'électrode triples (37), qui sont appliqués à distance fixe les uns des autres respectivement sur un support (29) commun ou un étrier (26) commun.

13. Appareil selon l'une quelconque des revendications précédentes, **caractérisé en ce qu'**il comprend un appareil à ECG.

14. Appareil selon la revendication 13, **caractérisé en ce que** les électrodes d'impédance (E1-18) peuvent être activées en partie également en tant qu'électrodes d'ECG (RA, LA, RL, LL, V1-V6, V5r, V6r).

15. Appareil selon l'une quelconque des revendications précédentes, **caractérisé en ce que** des relais électriques ou des commutateurs électroniques (23) sont prévus pour la commutation du courant en direction et en provenance des électrodes et pour la mesure de l'impédance.

16. Appareil selon l'une quelconque des revendications précédentes, **caractérisé en ce que** la fréquence du courant alternatif est modifiée en continu d'une fréquence de mesure inférieure à une fréquence de mesure supérieure.
